(19) Europäisches Patentamt
European Patent Office
Office européen des brevets

(11) EP 0 809 666 B2

(12) NEUE EUROPÄISCHE PATENTSCHRIFT

(45) Veröffentlichungstag und Bekanntmachung des Hinweises auf die Entscheidung über den Einspruch:
**14.08.2002 Patentblatt 2002/33**

(45) Hinweis auf die Patenterteilung:
**21.10.1998 Patentblatt 1998/43**

(21) Anmeldenummer: **96902980.0**

(22) Anmeldetag: **03.02.1996**

(51) Int Cl.[7]: **C08G 63/60**, C08G 63/672

(86) Internationale Anmeldenummer:
**PCT/EP96/00458**

(87) Internationale Veröffentlichungsnummer:
**WO 96/25448 (22.08.1996 Gazette 1996/38)**

(54) **BIOLOGISCH ABBAUBARE POLYMERE, VERFAHREN ZU DEREN HERSTELLUNG SOWIE DEREN VERWENDUNG ZUR HERSTELLUNG BIOABBAUBARER FORMKÖRPER**

BIODEGRADABLE POLYMERS, PROCESS FOR PRODUCING THEM AND THEIR USE IN PREPARING BIODEGRADABLE MOULDINGS

POLYMERES BIODEGRADABLES, LEUR PROCEDE DE FABRICATION ET LEUR EMPLOI DANS LA FABRICATION DE PIECES MOULEES BIODEGRADABLES

(84) Benannte Vertragsstaaten:
**BE CH DE DK ES FR GB IT LI NL SE**

(30) Priorität: **16.02.1995 DE 19505186**

(43) Veröffentlichungstag der Anmeldung:
**03.12.1997 Patentblatt 1997/49**

(73) Patentinhaber: **BASF AKTIENGESELLSCHAFT**
**67056 Ludwigshafen (DE)**

(72) Erfinder:
- **WARZELHAN, Volker**
  **D-67273 Weisenheim (DE)**
- **PIPPER, Gunter**
  **D-67098 Bad Dürkheim (DE)**
- **SEELIGER, Ursula**
  **D-67059 Ludwigshafen (DE)**
- **BAUER, Peter**
  **D-67071 Ludwigshafen (DE)**
- **BEIMBORN, Dieter, Bernhard**
  **D-67273 Weisenheim (DE)**
- **YAMAMOTO, Motonori**
  **D-68199 Mannheim (DE)**

(56) Entgegenhaltungen:

| | |
|---|---|
| **EP-A- 0 515 203** | **EP-A- 0 565 235** |
| **EP-A- 0 569 143** | **EP-A- 0 792 312** |
| **WO-A-92/09654** | **US-A- 3 763 079** |
| **US-A- 4 013 624** | **US-A- 4 252 940** |
| **US-A- 4 419 507** | **US-A- 4 966 959** |
| **US-A- 5 039 760** | **US-A- 5 070 180** |
| **US-A- 5 171 308** | |

- **DATABASE WPI Week 8142 Derwent Publications Ltd., London, GB; AN 81-76516D XP002004706 & JP,A,56 110 720 (TEIJIN KK) , 2.September 1981**
- **DATABASE WPI Week 9410 Derwent Publications Ltd., London, GB; AN 94-079832 XP002004707 & JP,A,06 032 357 (SHOWA HIGH POLYMER CO., LTD) , 8.Februar 1994**
- **H.Inata et al.: "Chain Extenders for Polyesters I." J.of Appl.Polym. Sci., Vol. 30, 3325-3337; 1985**
- **H.Inata et al.:"Chain Extenders for Polyesters II." J.of Appl. Polym. Sci., Vol. 32, 5193-5202; 1986**
- **Kirk-Othmer, Encycl. of Chem. Techn., 3rd edition, supplement vol., John Wiley & Sons, New York, 1984, p. 626-643**

EP 0 809 666 B2

Printed by Jouve, 75001 PARIS (FR)

## Beschreibung

Die vorliegende Erfindung betrifft biologisch abbaubare Polyetherester Q2 gemäß Anspruch 1.

Des weiteren betrifft die Erfindung Polymere und biologisch abbaubare thermoplastische Formmassen gemäß Unteransprüche, Verfahren zu deren Herstellung, deren Verwendung zur Herstellung biologisch abbaubarer Formkörper sowie Klebstoffe, biologisch abbaubare Formkörper, Schäume und Blends mit Stärke, erhältlich aus den erfindungsgemäßen Polymeren bzw. Formmassen.

Polymere, die biologisch abbaubar sind, d.h. die unter Umwelteinflüssen in einer angemessenen und nachweisbaren Zeitspanne zerfallen, sind seit einiger Zeit bekannt. Der Abbau erfolgt dabei in der Regel hydrolytisch und/oder oxidativ, zum überwiegenden Teil jedoch durch die Einwirkung von Mikroorganismen wie Bakterien, Hefen, Pilzen und Algen. Y. Tokiwa und T. Suzuki (Nature, Bd. 270, S. 76-78, 1977) beschreiben den enzymatischen Abbau von aliphatischen Polyestern, beispielsweise auch Polyester auf der Basis von Bernsteinsäure und aliphatischer Diole.

In der EP-A 565,235 werden aliphatische Copolyester, enthaltend [-NH-C(O)O-]-Gruppen ("Urethan-Einheiten"), beschrieben. Die Copolyester der EP-A 565,235 werden durch Umsetzung eines Präpolyesters - erhalten durch Umsetzung von im wesentlichen Bernsteinsäure und eines aliphatischen Diols - mit einem Diisocyanat, bevorzugt Hexamethylendiisocyanat, erhalten. Die Umsetzung mit dem Diisocyanat ist gemäß der EP-A 565,235 erforderlich, da durch die Polykondensation alleine nur Polymere mit solchen Molekulargewichten erhalten werden, die keine befriedigenden mechanischen Eigenschaften aufweisen. Von entscheidendem Nachteil ist die Verwendung von Bernsteinsäure oder deren Esterderivate zur Herstellung der Copolyester, weil Bernsteinsäure bzw. deren Derivate teuer und in nicht genügender Menge auf dem Markt verfügbar sind. Außerdem werden bei Verwendung von Bernsteinsäure als einziger Säurekomponente die daraus hergestellten Polyester nur extrem langsam abgebaut.

Aus der WO 92/13020 sind Copolyetherester auf Basis überwiegend aromatischer Dicarbonsäuren, kurzkettiger Etherdiol-Segmenten wie Diethylenglykol, langkettiger Polyalkylenglykole wie Polyethylenglykol (PEG) und aliphatischer Diole bekannt, wobei mindestens 85 mol-% des Polyesterdiolrestes aus einem Terephthalsäurerest bestehen. Durch Modifikationen wie den Einbau von bis zu 2,5 Mol-% Metallsalze der 5-Sulfoisophthalsäure kann die Hydrophilie des Copolyesters gesteigert und die Kristallinität vermindert werden. Hierdurch soll gemäß der WO 92/13020 ein biologischer Abbau der Copolyester ermöglicht werden. Nachteilig an diesen Copolyestern ist jedoch, daß ein biologischer Abbau durch Mikroorganismen nicht nachgewiesen wurde, sondern lediglich das Verhalten gegenüber Hydrolyse in kochendem Wasser durchgeführt wurde.

Nach Angaben von Y. Tokiwa und T. Suzuki (Nature, Bd. 270, 1977 oder J. of Appl. Polymer Science, Bd. 26, S. 441-448, 1981) ist davon auszugehen, daß Polyester, die weitgehend aus aromatischen Dicarbonsäure-Einheiten und aliphatischen Diolen aufgebaut sind, wie PET (Polyethylenterephthalat) und PBT (Polybutylenterephthalat), enzymatisch nicht abbaubar sind. Dies gilt auch für Copolyester und Copolyetherester, die Blöcke, aufgebaut aus aromatischen Dicarbonsäureeinheiten und aliphatischen Diolen bzw. Etherdiolen, enthalten.

Witt et al. (Handout zu einem Poster auf dem International Workshop des Royal Institute of Technology, Stockholm, Schweden, vom 21.bis 23.04.94) beschreiben biologisch abbaubare Copolyester auf der Basis von 1,3-Propandiol, Terephthalsäureester und Adipinoder Sebazinsäure. Nachteilig an diesen Copolyestern ist, daß daraus hergestellte Formkörper, insbesondere Folien, unzureichende mechanische Eigenschaften aufweisen.

WO 92/09654 offenbart aliphatisch-aromatische Copolyester, die biologisch abbaubar sein können, und aus 95-35 mol-% CO-C8-Alkyloder C2-C4-Oxyalkyldicarbonsäureresten, 5-65 mol-% C6-C10-Aryldicarbonsäureresten sowie C2-C8-Alkyl- oder -oxyalkyldiolresten aufgebaut sind. Die mechanischen Eigenschaften dieser Copolyester sind jedoch verbesserungswürdig.

Aufgabe der vorliegenden Erfindung war es daher, biologisch, d.h. durch Mikroorganismen, abbaubare Polymere bereitzustellen, die diese Nachteile nicht aufweisen. Insbesondere sollten die erfindungsgemäßen Polymere aus bekannten und preiswerten Monomerbausteinen herstellbar und wasserunlöslich sein. Des weiteren sollte es möglich sein, durch spezifische Modifikationen wie Kettenverlängerung, Einbau von hydrophilen Gruppen und verzweigend wirkenden Gruppen, maßgeschneiderte Produkte für die gewünschten erfindungsgemäßen Anwendungen zu erhalten. Dabei sollte der biologische Abbau durch Mikroorganismen nicht auf Kosten der mechanischen Eigenschaften erreicht werden, um die Zahl der Anwendungsgebiete nicht einzuschränken.

Demgemäß wurden die eingangs definierten Polymere und thermoplastischen Formmassen gefunden.

Des weiteren wurden Verfahren zu deren Herstellung, deren Verwendung zur Herstellung biologisch abbaubarer Formkörper und Klebstoffe sowie biologisch abbaubare Formkörper und Klebstoffe, erhältlich aus den erfindungsgemäßen Polymeren und Formmassen, gefunden.

Die Polyetherester P1 sind charakterisiert durch ein Molekulargewicht ($M_n$) im Bereich von 5000 bis 80000, vorzugsweise von 6000 bis 45000, besonders bevorzugt von 8000 bis 35000 g/mol, eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polyetherester P1 bei einer Temperatur von 25°C) und einen Schmelzpunkt im Bereich von 50 bis 200, vorzugsweise von 60 bis 160°C, sowie der weiteren Maßgabe, daß die Polyetherester P1

sowohl Hydroxyl- als auch Carboxylendgruppen besitzen, wobei man das Molverhältnis von Carboxylendgruppen zu Hydroxylendgruppen größer als eins, bevorzugt größer als zwei, wählt.

Die Polyetherester P1 erhält man, indem man eine Mischung, bestehend im wesentlichen aus

(a1) einer Mischung, bestehend im wesentlichen aus
20 bis 95, vorzugsweise von 30 bis 80, besonders bevorzugt von 40 bis 70 mol-% Adipinsäure oder esterbildende Derivate davon, insbesondere die Di-$C_1$-$C_6$-alkylester wie Dimethyl-, Diethyl-, Dipropyl-, Dibutyl-, Diisobutyl-, Dipentyl- und Dihexyladipat, oder deren Mischungen, bevorzugt Adipinsäure und Dimethyladipat, oder Mischungen davon,
5 bis 80, vorzugsweise 20 bis 70, besonders bevorzugt von 30 bis 60 mol-%, Terephthalsäure oder esterbildende Derivate davon, insbesondere die Di-$C_1$-$C_6$-alkylester wie Dimethyl-, Diethyl-, Dipropyl-, Dibutyl-, Dipentyl- oder Dihexylterephthalat, oder deren Mischungen, bevorzugt Terephthalsäure und Dimethylterephthalat, oder Mischungen davon, und
0 bis 5, vorzugsweise von 0 bis 3, besonders bevorzugt von 0,1 bis 2 mol-% einer sulfonatgruppenhaltigen Verbindung,
wobei die Summe der einzelnen Molprozentangaben 100 mol-% beträgt, und

(a2) einer Mischung aus Dihydroxyverbindungen, bestehend im wesentlichen aus

(a21) von 15 bis 99,8, vorzugsweise von 60 bis 99,5, besonders bevorzugt von 70 bis 99,5 mol-% einer Dihydroxyverbindung, ausgewählt aus der Gruppe bestehend aus $C_2$-$C_6$-Alkandiolen und $C_5$-$C_{10}$-Cycloalkandiolen,

(a22) von 85 bis 0,2, vorzugsweise von 0,5 bis 40, besonders bevorzugt von 0,5 bis 30 mol-% einer Etherfunktionenenthaltenden Dihydroxyverbindung gemäß Formel I

$$HO\text{-}[(CH_2)_n\text{-}O]_m\text{-}H \qquad I$$

in der n für 2, 3 oder 4, vorzugsweise zwei und drei, besonders bevorzugt zwei, und m für eine ganze Zahl von 2 bis 250, vorzugsweise von zwei bis 100 stehen, oder Mischungen davon,
wobei man das Molverhältnis von (a1) zu (a2) im Bereich von 0,4:1 bis 1,5:1, vorzugsweise von 0,6:1 bis 1,25:1 wählt, zur Reaktion bringt.

Als sulfonatgruppenhaltige Verbindung setzt man üblicherweise ein Alkali- oder Erdalkalimetallsalz einer sulfonatgruppenhaltigen Dicarbonsäure oder deren esterbildende Derivate ein, bevorzugt Alkalimetallsalze der 5-Sulphoisophthalsäure oder deren Mischungen, besonders bevorzugt das Natriumsalz.

Als Dihydroxyverbindungen (a21) setzt man eine Verbindung, ausgewählt aus der Gruppe bestehend aus $C_2$-$C_6$-Alkandiolen und $C_5$-$C_{10}$-Cycloalkandiolen, ein wie Ethylenglykol, 1,2-, 1,3-Propandiol, 1,2-, 1,4-Butandiol, 1,5-Pentandiol oder 1,6-Hexandiol, insbesondere Ethylenglykol, 1,3-Propandiol und 1,4-Butandiol, Cyclopentandiol, Cyclohexandiol, 1,2-Cyclohexandimethanol, 1,4-Cyclohexandimethanol, besonders bevorzugt Ethylenglykol und 1,4-Butandiol, sowie Mischungen daraus, ein.

Als Dihydroxyverbindungen (a22) setzt man bevorzugt Diethylenglykol, Triethylenglykol, Polyethylenglykol, Polypropylenglykol und Polytetrahydrofuran (Poly-THF), besonders bevorzugt Diethylenglykol, Triethylenglykol und Polyethylenglykol, ein, wobei man auch Mischungen davon oder Verbindungen, die unterschiedliche n's aufweisen (siehe Formel I), beispielsweise Polyethylenglykol, das Propyleneinheiten (n = 3) enthält, beispielsweise erhältlich durch Polymerisation nach an sich bekannten Methoden von zuerst Ethylenoxid und anschließend mit Propylenoxid, besonders bevorzugt ein Polymer auf Basis von Polyethylenglykol, mit unterschiedlichen n's, wobei Einheiten gebildet aus Ethylenoxid überwiegen. Das Molekulargewicht ($M_n$) des Polyethylenglykols wählt man in der Regel im Bereich von 250 bis 8000, bevorzugt von 600 bis 3000 g/mol.

Man verwendet von 0,01 bis 4 mol-%, besonders bevorzugt von 0,05 bis 4 mol-%, bezogen auf die Komponente (a1), mindestens eine Verbindung D mit mindestens drei zur Esterbildung befähigten Gruppen.

Die Verbindungen D enthalten bevorzugt drei bis zehn funktionelle Gruppen, welche zur Ausbildung von Esterbindungen fähig sind. Besonders bevorzugte Verbindungen D haben drei bis sechs funktionelle Gruppen dieser Art im Molekül, insbesondere drei bis sechs Hydroxylgruppen und/oder Carboxylgruppen. Beispielhaft seien genannt:

Weinsäure, Citronensäure, Äpfelsäure;
Trimethylolpropan, Trimethylolethan;
Pentaerythrit;

Polyethertriole;
Glycerin;
Trimesinsäure;
Trimellitsäure, -anhydrid;
Pyromellitsäure, -dianhydrid und
Hydroxyisophthalsäure.

Beim Einsatz von Verbindungen D, die einen Siedepunkt unterhalb von 200°C aufweisen, kann bei der Herstellung der Polymerester P1 ein Anteil vor der Reaktion aus dem Polykondensationsgemisch abdestillieren. Es ist daher bevorzugt, diese Verbindungen in einer frühen Verfahrensstufe wie der Umesterungs- bzw. Veresterungsstufe zuzusetzen, um diese Komplikation zu vermeiden und um die größtmögliche Regelmäßigkeit ihrer Verteilung innerhalb des Polykondensats zu erzielen.

Im Falle höher als 200°C siedender Verbindungen D können diese auch in einer späteren Verfahrensstufe eingesetzt werden.

Durch Zusatz der Verbindung D kann beispielsweise die Schmelzviskosität in gewünschter Weise verändert, die Schlagzähigkeit erhöht und die Kristallinität der erfindungsgemäßen Polymere bzw. Formmassen herabgesetzt werden.

Die Herstellung der biologisch abbaubaren Polyetherester P1 ist grundsätzlich bekannt (Sorensen und Campbell, "Preparative Methods of Polymer Chemistry", Interscience Publishers, Inc., New York, 1961, Seiten 111 bis 127; Encyl. of Polym. Science and Eng., Bd. 12, 2. Ed., John Wiley & Sons, 1988, S. 75 bis 117; Kunststoff-Handbuch, Band 3/1, Carl Hanser Verlag, München, 1992, S. 15 bis 23 (Herstellung von Polyeltern); WO 92/13020; EP-A 568,593; EP-A 565,235; EP-A 28,687), so daß sich nähere Angaben hierüber erübrigen.

So kann man beispielsweise die Umsetzung von Dimethylestern der Komponente (a1) mit der Komponente (a2) ("Umesterung") bei Temperaturen im Bereich von 160 bis 230°C in der Schmelze bei Atmosphärendruck vorteilhaft unter Inertgasatmosphäre durchführen.

Vorteilhaft wird bei der Herstellung des biologisch abbaubaren Polyetheresters P1 ein molarer Überschuß der Komponente (a2), bezogen auf die Komponente (a1), verwendet, beispielsweise bis zum 2 1/2fachen, bevorzugt bis zum 1,67fachen.

Üblicherweise erfolgt die Herstellung des biologisch abbaubaren Polyetheresters P1 unter Zugabe von geeigneten, an sich bekannten Katalysatoren wie Metallverbindungen auf der Basis folgender Elemente wie Ti, Ge, Zn, Fe, Mn, Co, Zr, V, Ir, La, Ce, Li, und Ca, bevorzugt metallorganische Verbindungen auf der Basis dieser Metalle wie Salze organischer Säuren, Alkoxide, Acetylacetonate und ähnliches, insbesondere bevorzugt auf Basis von Zink, Zinn und Titan.

Bei Verwendung von Dicarbonsäuren oder deren Anhydride als Komponente (a1) kann deren Veresterung mit Komponente (a2) vor, gleichzeitig oder nach der Umesterung stattfinden. Beispielsweise kann das in der DE-A 23 26 026 beschriebene Verfahren zur Herstellung modifizierter Polyalkylenterephthalate verwendet werden.

Nach der Umsetzung der Komponenten (a1) und (a2) wird in der Regel unter vermindertem Druck oder in einem Inertgasstrom, beispielsweise aus Stickstoff, bei weiterem Erhitzen auf eine Temperatur im Bereich von 180 bis 260°C die Polykondensation bis zum gewünschten Molekulargewicht unter Berücksichtigung des Molverhältnisses der Carboxylendgruppen zu Hydroxylendgruppen, das man größer als eins, vorzugsweise größer als zwei, wählt, durchgeführt.

Das gewünschte Endgruppenverhältnis kann man einstellen

- durch einen entsprechenden Überschuß der Komponente a1,
- durch eine entsprechend lange Polykondensationszeit unter gleichzeitiger Entfernung des Diols bei einem Überschuß an Komponente a2, oder
- durch Zugabe einer entsprechenden Menge an polyfunktionellen Carbonsäuren oder deren Derivate, bevorzugt Dicarbonsäureanhydride wie Bernsteinsäureanhydrid, Phthalsäureanhydrid, Pyromellitsäureanhydrid oder Trimetllitsäureanhydrid, wenn Polyetherester P1 durch Einsatz eines Überschusses an Komponente a2 überwiegend Hydroxylendgruppen aufweist.

Um unerwünschte Abbau- und/oder Nebenreaktionen zu vermeiden, kann man in dieser Verfahrensstufe gewünschtenfalls auch Stabilisatoren (siehe EP-A 21042 und US-A 4,321,341) zusetzen. Solche Stabilisatoren sind beispielsweise die in der EP-A 13 461, US 4,328,049 oder in B. Fortunato et al., Polymer Vol. 35, Nr. 18, S. 4006 bis 4010, 1994, Butterworth-Heinemann Ltd., beschriebene Phosphor-Verbindungen. Diese können zum Teil auch als Deaktivatoren der oben beschriebenen Katalysatoren wirken. Beispielhaft seien genannt: Organophosphite, phosphonige Säure und phosphorige Säure. Als Verbindungen, die nur als Stabilisatoren wirken seien beispielhaft genannt: Trialkylphosphite, Triphenylphosphit, Trialkylphosphate, Triphenylphosphat und Tocopherol (beispielsweise als Uvinul® 2003AO (BASF) erhältlich).

Bei der Verwendung der erfindungsgemäßen biologisch abbaubaren Copolymere, beispielsweise im Verpackungsbereich z.B. für Nahrungsmittel, ist es in der Regel wünschenswert, den Gehalt an eingesetztem Katalysator so gering als möglich zu wählen sowie keine toxischen Verbindungen einzusetzen. Im Gegensatz zu anderen Schwermetallen wie Blei, Zinn, Antimon, Cadmium, Chrom etc. sind Titan- und Zinkverbindungen in der Regel nicht toxisch ("Sax Toxic Substance Data Book", Shizuo Fujiyama, Maruzen, K.K., 360 S. (zitiert in EP-A 565,235), siehe auch Römpp Chemie Lexikon Bd. 6, Thieme Verlag, Stuttgart, New York, 9. Auflage, 1992, S. 4626 bis 4633 und 5136 bis 5143). Beispielhaft seien genannt: Dibutoxydiacetoacetoxytitan, Tetrabutylorthotitanat und Zink(II)-acetat.

Das Gewichtsverhältnis von Katalysator zu biologisch abbaubaren Polyetherester P1 liegt üblicherweise im Bereich von 0,01:100 bis 3:100, vorzugsweise von 0,05:100 bis 2:100, wobei bei hochaktiven Titanverbindungen auch kleinere Mengen eingesetzt werden können wie 0,0001:100.

Der Katalysator kann gleich zu Beginn der Reaktion, unmittelbar kurz vor der Abtrennung des überschüssigen Diols oder gewünschtenfalls auch in mehreren Portionen verteilt während der Herstellung der biologisch abbaubaren Polyetherester P1 eingesetzt werden. Gewünschtenfalls können auch verschiedene Katalysatoren oder auch Gemische davon eingesetzt werden.

Die biologisch abbaubaren Polyetherester P2 sind charakterisiert durch ein Molekulargewicht ($M_n$) im Bereich von 5000 bis 80000, vorzugsweise von 6000 bis 45000, besonders vorzugsweise von 10000 bis 40000 g/mol, eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polyetherester P2 bei einer Temperatur von 25°C) und einen Schmelzpunkt im Bereich von 50 bis 235, vorzugsweise von 60 bis 235°C, und besitzen sowohl Hydroxyl- als auch Carboxylendgruppen, wobei man das Molverhältnis von Carboxylendgruppen zu Hydroxylendgruppen größer als eins, vorzugsweise größer als zwei, wählt.

Die biologisch abbaubaren Polyetherester P2 erhält man, indem man eine Mischung zur Reaktion bringt, bestehend im wesentlichen aus

(b1) einer Mischung, bestehend im wesentlichen aus
20 bis 95, bevorzugt von 25 bis 80, besonders bevorzugt von 30 bis 70 mol-% Adipinsäure oder esterbildende Derivate davon oder Mischungen davon,
5 bis 80, bevorzugt von 20 bis 75, besonders bevorzugt von 30 bis 70 mol-% Terephthalsäure oder esterbildende Derivate davon oder Mischungen davon, und
0 bis 5, bevorzugt von 0 bis 3, besonders bevorzugt von 0,1 bis 2 mol-% einer sulfonatgruppenhaltigen Verbindung,
wobei die Summe der einzelnen Molprozentangaben 100 mol-% beträgt,

(b2) einer Mischung aus Dihydroxyverbindungen (a2),
wobei man das Molverhältnis von (b1) zu (b2) im Bereich von 0,4:1 bis 1,25:1, vorzugsweise von 0,6:1 bis 1,25:1 wählt,

(b3) von 0,01 bis 100, vorzugsweise von 0,1 bis 80 Gew.-%, bezogen auf Komponente (b1), einer Hydroxycarbonsäure B1, und

(b4) von 0,01 bis 3,5 mol-%, bezogen auf Komponente (b1), Verbindung D,
wobei die Hydroxycarbonsäure B1 definiert ist durch die Formeln IIa oder IIb

$$HO-[-C(O)-G-O-]_pH$$

IIa

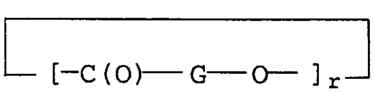

IIb

in der p eine ganze Zahl von 1 bis 1500, vorzugsweise von 1 bis 1000 und r 1, 2, 3 oder 4, vorzugsweise 1 und 2, bedeuten, und G für einen Rest steht, der ausgewählt ist aus der Gruppe bestehend aus Phenylen, $-(CH_2)_k-$, wobei k eine ganze Zahl von 1, 2, 3, 4 oder 5, vorzugsweise 1 und 5, bedeutet, $-C(R)H-$ und $-C(R)HCH_2$, wobei R für Methyl oder Ethyl steht.

Die Herstellung der biologisch abbaubaren Polyetherester P2 erfolgt zweckmäßig analog zur Herstellung der Polyetherester P1, wobei die Zugabe der Hydroxycarbonsäure B1 sowohl zu Anfang der Umsetzung als auch nach der Veresterungs- bzw. Umesterungsstufe erfolgen kann.

In einer bevorzugten Ausführungsform setzt man der Hydroxycarbonsäure B1 ein wie Glycolsäure, D-, L-, D, L-Milchsäure, 6-Hydroxyhexansäure, deren cyclische Derivate wie Glycolid (1,4-Dioxan-2,5-dion), D-, L-Dilactid (3,6-dimethyl-1,4-dioxan- 2,5-dion), p-Hydroxybenzoesäure sowie Oligomere und Polymere wie 3-Polyhydroxybuttersäure, Polyhydroxyvaleriansäure, Polylactid (beispielsweise als EcoPLA® (Fa. Cargill) erhältlich) sowie eine Mischung aus 3-Polyhydroxybuttersäure und Polyhydroxyvaleriansäure (beispielsweise unter dem Namen Biopol® von Zeneca erhältlich), besonders bevorzugt für die Herstellung von Polyetherester P2 die niedermolekularen und cyclischen Derivate davon.

Die biologisch abbaubaren Polyetherester Q1 sind charakterisiert durch ein Molekulargewicht ($M_n$) im Bereich von 5000 bis 100000, vorzugsweise von 8000 bis 80000, durch eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polyetherester Q1 bei einer Temperatur von 25°C), und einen Schmelzpunkt im Bereich von 50 bis 235, vorzugsweise von 60 bis 235°C, und besitzen sowohl Hydroxylals auch Carboxylendgruppen, wobei man das Molverhältnis von Carboxylendgruppen zu Hydroxylendgruppen größer als eins, vorzugsweise größer als zwei, wählt.

Die Polyetherester Q1 erhält man, indem man eine Mischung zur Reaktion bringt, bestehend im wesentlichen aus

(c1) Polyetherester P1,

(c2) 0,01 bis 50, vorzugsweise von 0,1 bis 40 Gew.-%, bezogen auf (c1), Hydroxycarbonsäure B1, und

(c3) 0 bis 5, vorzugsweise von 0 bis 4 mol-%, bezogen auf Komponente (a1) aus der Herstellung von P1, Verbindung D.

Die Umsetzung der Polyetherester P1 mit der Hydroxycarbonsäure B1 gewünschtenfalls in Gegenwart der Verbindung D erfolgt vorzugsweise in der Schmelze bei Temperaturen im Bereich von 120 bis 260°C unter Inertgasatmosphäre, gewünschtenfalls auch unter vermindertem Druck. Man kann sowohl diskontinuierlich als auch kontiriuierlich, beispielsweise in Rührkesseln oder (Reaktions-)Extrudern, arbeiten.

Die Umsetzung kann gewünschtenfalls durch Zugabe an sich bekannter Umesterungskatalysatoren (siehe die weiter oben bei der Herstellung der Polyetherester P1 beschriebenen) beschleunigt werden.

Eine bevorzugte Ausführungsform betrifft Polyetherester Q1 mit Blockstrukturen gebildet aus den Komponenten P1 und B1: bei Verwendung cyclischer Derivate von B1 (Verbindungen IIb) können bei der Umsetzung mit dem biologisch abbaubaren Polyetherester P1 durch eine sogenannte "ringöffnende Polymerisation", ausgelöst durch die Endgruppen von P1, in an sich bekannter Weise Polyetherester Q1 mit Blockstrukturen erhalten werden (zur "ringöffnenden Polymerisation" siehe Encycl. of Polym. Science and Eng. Bd. 12, 2.Ed., John Wiley & Sons, 1988, S. 1 bis 75, insbesondere S. 36 bis 41). Die Reaktion kann man gewünschtenfalls unter Zusatz üblicher Katalysatoren wie den bereits weiter oben beschriebenen Umesterungskatalysatoren durchführen, insbesondere bevorzugt ist Zinnoctanoat (siehe auch Encycl. of Polym. Science and Eng. Bd. 12, 2.Ed., John Wiley & Sons, 1988, S. 1 bis 75, insbesondere S.36 bis 41).

Bei Verwendung von Komponenten B1 mit höheren Molekulargewichten, beispielsweise mit einem p von größer als 10 (zehn), können durch Umsetzung mit den Polyetherestern P1 in Rührkesseln oder Extrudern, die gewünschten Blockstrukturen durch die Wahl der Reaktionsbedingungen wie Temperatur, Verweilzeit, Zusatz von Umesterungskatalysatoren wie den oben genannten erhalten werden. So ist aus J. of Appl. Polym. Sci., Vol. 32, S. 6191 bis 6207, John Wiley & Sons, 1986 sowie aus Makromol. Chemie, Vol. 136, S. 311 bis 313, 1970 bekannt, daß bei der Umsetzung von Polyetherestern in der Schmelze aus einem Blend durch Umesterungsreaktionen zunächst Blockcopolymere und dann statistische Copolymere erhalten werden können.

Die erfindungsgemäßen biologisch abbaubaren Polyetherester Q2 sind charakterisiert durch ein Molekulargewicht ($M_n$) im Bereich von 6000 bis 80000, vorzugsweise von 8000 bis 50000, besonders bevorzugt von 10000 bis 40000 g/mol, durch eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polyetherester Q2 bei einer Temperatur von 25°C), und einen Schmelzpunkt im Bereich von 50 bis 200°C, vorzugsweise von 60 bis 160°C, und besitzen sowohl Hydroxyl- als auch Carboxylendgruppen, wobei man das Molverhältnis von Carboxylendgruppen zu Hydroxylendgruppen größer als eins, vorzugsweise größer als zwei, wählt.

Die Polyetherester Q2 erhält man erfindungsgemäß, indem man eine Mischung zur Reaktion bringt, bestehend im wesentlichen aus

(d1) von 95 bis 99,9, vorzugsweise von 96 bis 99,8, besonders bevorzugt von 97 bis 99,65 Gew.-% Polyetherester P1,

(d2) von 0,1 bis 5, vorzugsweise 0,2 bis 4, besonders bevorzugt von 0,35 bis 3 Gew.-% eines Bisoxazolins C1 und

(d3) von 0 bis 5, vorzugsweise von 0 bis 4 mol-%, bezogen auf Komponente (a1) aus der Herstellung von P1, Verbindung D.

Als Bisoxazoline C1 kann man nach bisherigen Beobachtungen alle gebräuchlichen Bisoxazoline einsetzen. Entsprechende Bisoxazoline sind beispielsweise in der DE-A 39 15 874 beschrieben (unter der Bezeichnung Loxamid® im Handel erhältlich). Weitere Bisoxazoline sind in der WO 94/03523 (PCT/EP 93/01986) beschrieben.
Besonders bevorzugte Bisoxazoline C1 sind Bisoxazoline der allgemeinen Formel III

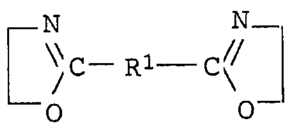

III

Die Bisoxazoline C1 der allgemeinen Formel III (Komponente d2) sind im allgemeinen erhältlich durch das Verfahren aus Angew. Chem. Int. Edit., Vol. 11 (1972), S. 287-288. Besonders bevorzugte Bisoxazoline sind solche, in denen $R^1$ eine Einfachbindung, eine $(CH_2)_q$-Alkylengruppe, mit q = 2, 3 oder 4 wie Methylen, Ethan-1,2-diyl, Propan-1,3-diyl, Propan-1,2-diyl, Butan-1,4-diyl oder eine Phenylengruppe bedeutet. Als insbesonders bevorzugte Bisoxazoline seien 2,2'-Bis(2-oxazolin), Bis (2-oxazolinyl)methan, 1,2-Bis(2-oxazolinyl)ethan, 1,3-Bis(2-oxazolinyl)propan, 1,4-Bis(2-oxazolinyl)butan, 1,4-Bis(2-oxazolinyl)benzol, 1,2-Bis(2-oxazolinyl)benzol und 1,3-Bis(2-oxazolinyl)benzol genannt.
Die Umsetzung der Polyetherester P1 mit dem Bisoxazolin C1 erfolgt vorzugsweise in der Schmelze (siehe auch: J. Appl. Polym. Science, Vol. 33, S. 3069-3079 (1987)), wobei darauf zu achten ist, daß möglichst keine Nebenreaktionen auftreten, die zu einer Vernetzung oder Gelbildung führen können. In einer besonderen Ausführungsform führt man die Reaktion üblicherweise bei Temperaturen im Bereich von 120 bis 260, vorzugsweise von 130 bis 240, besonders bevorzugt 140 bis 220°C durch, wobei die Zugabe des Bisoxazolins vorteilhaft in mehreren Portionen oder kontinuierlich erfolgt.
Gewünschtenfalls kann man die Umsetzung der Polyetheresters P1 mit dem Bisoxazolins C1 auch in Gegenwart von gängigen inerten Lösemitteln wie Toluol, Methylethylketon oder Dimethylformamid ("DMF") oder deren Mischungen durchführen, wobei man die Reaktionstemperatur in der Regel im Bereich von 80 bis 200, vorzugsweise von 90 bis 150°C wählt.
Die Umsetzung mit dem Bisoxazolin C1 kann diskontinuierlich oder kontinuierlich beispielsweise in Rührkesseln, Reaktionsextrudern oder über Mischköpfe durchgeführt werden.
Obwohl das theoretische Optimum für die Reaktion von P1 mit Bisoxazolinen C1 bei einem Molverhältnis der Oxazolin-Funktion zu P1-Carboxylendgruppe (besonders bevorzugt sind Polyetherester P1 mit überwiegend Carboxylendgruppen) von 1:1 liegt, kann die Umsetzung ohne technische Probleme auch bei Molverhältnissen von 1:3 bis 1,5: 1 durchgeführt werden. Bei den erfindungsgemäßen Molverhältnissen von >1:1, vorzugsweise >2:1, kann gewünschtenfalls während der Umsetzung oder auch nach der Umsetzung die Zugabe einer Dicarbonsäure, bevorzugt ausgewählt aus der Gruppe aus Adipinsäure, Bernsteinsäure, Terephthalsäure und Isophthalsäure, erfolgen.
Die erfindungsgemäßen biologisch abbaubaren Polymere T1 sind charakterisiert durch ein Molekulargewicht ($M_n$) im Bereich von 10000 bis 100000, vorzugsweise von 11000 bis 80000, vorzugsweise von 11000 bis 50000 g/mol, mit einer Viskositätszahl im Bereich von 30 bis 450, vorzugsweise von 50 bis 400 g/ml (gemessen in o-Dichlorbenzol/ Phenol (Gew.-Verhältnis 50/50) bei einer Konzentration von 0,5 Gew.-% Polymer T1 bei einer Temperatur von 25°C) und einem Schmelzpunkt im Bereich von 50 bis 235, vorzugsweise von 60 bis 235°C.
Die biologisch abbaubaren Polymere T1 erhält man erfindungsgemäß, indem man einen Polyetherester Q1 gemäß Anspruch 3 mit

(e1) 0,1 bis 5, vorzugsweise von 0,2 bis 4, besonders bevorzugt von 0,3 bis 3 Gew.-%, bezogen auf den Polyetherester Q1, Bisoxazolin C1 sowie mit

(e2) 0 bis 5, vorzugsweise von 0 bis 4 mol-%, bezogen auf Komponente (a1) aus der Herstellung von P1 sowie Polyetherester Q1, Verbindung D zur Reaktion bringt.

Auf diese Weise wird üblicherweise eine Kettenverlängerung erreicht, wobei die erhaltenen Polymerketten vorzugsweise eine Blockstruktur aufweisen.

Die Umsetzung erfolgt in der Regel analog zur Herstellung der Polyetherester Q2.

Die erfindungsgemäßen biologisch abbaubaren Polymere T2 sind charakterisiert durch ein Molekulargewicht ($M_n$) im Bereich von 10000 bis 100000, vorzugsweise von 11000 bis 80000, besonders bevorzugt von 11000 bis 50000 g/mol, mit einer viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polymer T2 bei einer Temperatur von 25 °C) und einem Schmelzpunkt im Bereich von 50 bis 235, vorzugsweise von 60 bis 235°C.

Die biologisch abbaubaren Polymere T2 erhält man erfindungsgemäß durch Umsetzung des Polyetheresters Q2 mit

(f1) 0,01 bis 50, vorzugsweise von 0,1 bis 40 Gew.-%, bezogen auf den Polyetherester Q2, der Hydroxycarbonsäure B1 sowie mit

(f2) 0 bis 5, vorzugsweise von 0 bis 4 mol-%, bezogen auf Komponente (a1 aus der Herstellung von Polyetherester Q2 über den Polyetherester P1, Verbindung D,
wobei man zweckmäßig analog zur Umsetzung von Polyetherester P1 mit Hydroxycarbonsäure B1 zu Polyetherester Q1 verfährt.

Die erfindungsgemäßen biologisch abbaubaren Polymere T3 sind charakterisiert durch ein Molekulargewicht ($M_n$) im Bereich von 10000 bis 100000, vorzugsweise von 11000 bis 80000 g/mol, eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polymer T3 bei einer Temperatur von 25°C) und einem Schmelzpunkt im Bereich von 50 bis 235, vorzugsweise von 60 bis 235°C.

Die biologisch abbaubaren Polymere T3 erhält man erfindungsgemäß, indem man (g1) Polyetherester P2, oder (g2) einer Mischung bestehend im wesentlichen aus Polyetherester P1 und 0,01 bis 50, vorzugsweise von 0,1 bis 40 Gew.-%, bezogen auf den Polyetherester P1, Hydroxycarbonsäure B1, oder (g3) einer Mischung, bestehend im wesentlichen aus Polyetherestern P1, die eine unterschiedliche Zusammensetzung voneinander aufweisen, mit
0,1 bis 5, vorzugsweise von 0,2 bis 4, besonders bevorzugt von 0,3 bis 2,5 Gew.-%, bezogen auf die Menge der eingesetzten Polyetherester, Bisoxazolin C1 sowie
mit 0 bis 5, vorzugsweise von 0 bis 4 mol-%, bezogen auf die jeweiligen Molmengen an Komponente (a1), die zur Herstellung der eingesetzten Polyetherester (g1) bis (g3) eingesetzt wurden, Verbindung D, zur Reaktion bringt, wobei man die Umsetzungen zweckmäßig analog zur Herstellung der Polyetherester Q2 aus den Polyetherestern P1 und den Bisoxazolinen C1 vornimmt.

In einer bevorzugten Ausführungsform setzt man Polyetherester P2 ein, deren wiederkehrende Einheiten statistisch im Molekül verteilt sind.

Man kann jedoch auch Polyetherester P2 einsetzen, deren Polymerketten Blockstrukturen aufweisen. Solche Polyetherester P2 sind im allgemeinen zugänglich durch entsprechende Wahl, insbesondere des Molekulargewichts, der Hydroxycarbonsäure B1. So erfolgt nach bisherigen Beobachtungen im allgemeinen bei Verwendung einer hochmolekularen Hydroxycarbonsäure B1, insbesondere mit einem p von größer als 10, nur eine unvollständige Umesterung, beispielsweise auch in Gegenwart der oben beschriebenen Deaktivatoren (siehe J. of Appl. Polym. Sc. Vol. 32, S. 6191 bis 6207, John Wiley & Sons, 1986, und Makrom. Chemie, Vol. 136, S. 311 bis 313, 1970). Gewünschtenfalls kann man die Umsetzung auch in Lösung mit den bei der Herstellung der Polymeren T1 aus den Polyetherestern Q1 und den Bisoxazolinen C1 genannten Lösungsmitteln durchführen.

Die biologisch abbaubaren thermoplastischen Formmassen T4 erhält man erfindungsgemäß, indem man in an sich bekannter Weise, bevorzugt unter Zusatz üblicher Additive wie Stabilisatoren, Verarbeitungshilfsmitteln, Füllstoffen etc. (siehe J. of Appl. Polym. Sc., Vol. 32, S. 6191 bis 6207, John Wiley & Sons, 1986; WO 92/0441; EP 515,203; Kunststoff-Handbuch, Bd. 3/1, Carl Hanser Verlag München, 1992, S. 24 bis 28)

(h1) 99,5 bis 0,5 Gew.-% Polyetherester P1 gemäß Anspruch 1 oder Polyetherester Q2 gemäß Anspruch 4 mit

(h2) 0,5 bis 99,5 Gew.-% Hydroxycarbonsäure B1 mischt.

In einer bevorzugten Ausführungsform setzt man hochmolekulare Hydroxycarbonsäuren B1 wie Polycaprolacton oder Polylactid (z.B. EcoPLA®) oder Polyglykolid oder Polyhydroxyalkanoate wie 3-Polyhydroxybuttersäure, Polyhydroxyvaleriansäure sowie deren Mischungen (z.B. Biopol®), mit einem Molekulargewicht ($M_n$) im Bereich von 10000 bis 150000, vorzugsweise von 10000 bis 100000 g/mol ein.

Aus WO 92/0441 und EP-A 515,203 ist es bekannt, daß hochmolekulares Polylactid ohne Zusätze von Weichmachern für die meisten Anwendungen zu spröde ist. In einer bevorzugten Ausführungsform kann man ein Blend ausgehend von 0,5 bis 20, vorzugsweise von 0,5 bis 10 Gew.-% Polyetherester P1 gemaß Anspruch 1 oder Polyetherester Q2 gemäß Anspruch 4 und

99,5 bis 80, vorzugsweise von 99,5 bis 90 Gew.-% Polylactid herstellen, das eine deutliche Verbesserung der mechanischen Eigenschaften, beispielsweise eine Erhöhung der Schlagzähigkeit, gegenüber reinem Polylactid aufweist.

Eine weitere bevorzugte Ausführungsform betrifft ein Blend, erhältlich durch Mischen

von 99,5 bis 40, vorzugsweise von 99,5 bis 60 Gew.-% Polyetherester P1 gemäß Anspruch 1 oder Polyetherester Q2 gemäß Anspruch 4 und

von 0,5 bis 60, vorzugsweise von 0,5 bis 40 Gew.-% einer hochmolekularen Hydroxycarbonsäure B1 besonders bevorzugt Polylactid (z.B. EcoPLA®), Polyglycolid, 3-Polyhydroxybuttersäure, Polyhydroxyvaleriansäure sowie deren Mischungen (z.B. Biopol®), und Polycaprolacton. Solche Blends können vollständig biologisch abgebaut werden und weisen nach den bisherigen Beobachtungen sehr gute mechanische Eigenschaften auf.

Nach bisherigen Beobachtungen erhält man die erfindungsgemäßen thermoplastischen Formmassen T4 bevorzugt dadurch, daß man kurze Mischzeiten einhält, beispielsweise bei einer Durchführung des Mischens in einem Extruder. Durch Wahl der Mischparameter, insbesondere der Mischzeit und gewünschtenfalls der Verwendung von Deaktivatoren, sind auch Formmassen zugänglich, die überwiegend Blendstrukturen aufweisen, d.h., daß der Mischvorgang so gesteuert werden kann, daß zumindest teilweise auch Umesterungsreaktionen stattfinden können.

In einer weiteren bevorzugten Ausführungsform kann man O bis 50, vorzugsweise O bis 30 Mol-% der Adipinsäure, oder ihrer esterbildende Derivate oder deren Mischungen, durch mindestens eine andere aliphatische $C_4$-$C_{10}$- oder cycloaliphatische $C_5$-$C_{10}$-Dicarbonsäure oder Dimerfettsäure wie Bernsteinsäure, Glutarsäure, Pimelinsäure, Korksäure, Azelainsäure oder Sebazinsäure oder ein Esterderivat wie deren Di-$C_1$-$C_6$-alkylester oder deren Anhydride wie Bernsteinsäureanhydrid, oder deren Mischungen, ersetzen, bevorzugt Bernsteinsäure, Bernsteinsäureanhydrid, Sebacinsäure, Dimerfettsäure und Di-$C_1$-$C_6$-alkylester wie Dimethyl-, Diethyl-, Di-n-Propyl-, Diisobutyl-, Di-n-pentyl-, Dineopentyl-, Di-n-hexyl-ester davon, insbesondere Dimethylbernsteinsäure.

Eine besonders bevorzugte Ausführungsform betrifft den Einsatz als Komponente (a1) die in der EP-A 7445 beschriebene Mischung aus Bernsteinsäure, Adipinsäure und Glutarsäure sowie deren $C_1$-$C_6$-Alkylester wie Dimethyl-, Diethyl-, Di-n-Propyl-, Diisobutyl-, Di-n-pentyl-, Dineopentyl-, Di-n-hexyl-ester, insbesondere deren Dimethylester und Diisobutylester.

In einer weiteren bevorzugten Ausführungsform kann man O bis 50, vorzugsweise O bis 40 Mol-% der Terephthalsäure oder ihrer esterbildende Derivate, oder deren Mischungen durch mindestens eine andere aromatische Dicarbonsäure wie Isophthalsäure, Phthalsäure oder 2,6-Naphthalindicarbonsäure, bevorzugt Isophthalsäure, oder ein Esterderivat wie einen Di-$C_1$-$C_6$-alkylester wie Dimethyl-, Diethyl-, Di-n-Propyl-, Diisobutyl-, Di-n-pentyl-, Dineopentyl-, Di-n-hexyl-ester, insbesondere einen Dimethylester, oder deren Mischungen, ersetzen.

Allgemein sei angemerkt, daß man die unterschiedlichen erfindungsgemäßen Polymere wie üblich aufarbeiten kann, indem man die Polymere isoliert, oder, insbesondere, wenn man die Polyetherester P1, P2, Q1 und Q2 weiter umsetzen möchte, indem man die Polymere nicht isoliert, sondern gleich weiterverarbeitet.

Die erfindungsgemäßen Polymere kann man durch Walzen, Streichen, Spritzen oder Gießen auf Beschichtungsunterlagen aufbringen. Bevorzugte Beschichtungsunterlagen sind solche, die kompostierbar sind oder verrotten wie Formkörper aus Papier, Cellulose oder Stärke.

Die erfindungsgemäßen Polymere können außerdem zur Herstellung von Formkörpern verwendet werden, die kompostierbar sind. Als Formkörper seien beispielhaft genannt: Wegwerfgegenstände wie Geschirr, Besteck, Müllsäcke, Folien für die Landwirtschaft zur Ernteverfrühung, Verpackungsfolien und Gefäße für die Anzucht von Pflanzen.

Des weiteren kann man die erfindungsgemäßen Polymere in an sich bekannter Weise zu Fäden verspinnen. Die Fäden kann man gewünschtenfalls nach üblichen Methoden verstrecken, streckzwirnen, streckspulen, streckschären, streckschlichten und strecktexturieren. Die Verstreckung zu sogenanntem Glattgarn kann dabei in ein und demselben Arbeitsgang (fully drawn yarn oder fully oriented yarn), oder in einem getrennten Arbeitsgang erfolgen. Das Streckschären, Streckschlichten und die Strecktexturierung führt man im allgemeinen in einem vom Spinnen getrennten Arbeitsgang durch. Die Fäden kann man in an sich bekannter Weise zu Fasern weiterverarbeiten. Aus den Fasern sind dann Flächengebilde durch Weben, Wirken oder Stricken zugänglich.

Die oben beschriebenen Formkörper, Beschichtungsmittel und Fäden etc. können gewünschtenfalls auch Füllstoffe enthalten, die man während des Polymerisationsvorganges in irgendeiner Stufe oder nachträglich, beispielsweise in eine Schmelze der erfindungsgemäßen Polymere einarbeiten kann.

Bezogen auf die erfindungsgemäßen Polymere kann von 0 bis 80 Gew.-% Füllstoffe zusetzen. Geeignete Füllstoffe sind beispielsweise Ruß, Stärke, Ligninpulver, Cellulosefasern, Naturfasern wie Sisal und Hanf, Eisenoxide, Tonmineralien, Erze, Calciumcarbonat, Calciumsulfat, Bariumsulfat und Titandioxid. Die Füllstoffe können zum Teil auch Stabilisatoren wie Tocopherol (Vitamin E), organische Phosphorverbindungen, Mono-, Di- und Polyphenole, Hydrochinone, Diarylamine, Thioether, UV-Stabilisatoren, Nukleierungsmittel wie Talkum sowie Gleit- und Formtrennmittel auf Basis von Kohlenwasserstoffen, Fettalkoholen, höheren Carbonsäuren, Metallsalzen höherer Carbonsäuren

wie Calcium- und Zinkstearat, und Montanwachsen enthalten. Solche Stabilisatoren etc. sind in Kunststoff-Handbuch, Bd. 3/1, Carl Hanser Verlag, München, 1992, S. 24 bis 28 ausführlich beschrieben.

Die erfindungsgemäßen Polymere können außerdem durch den Zusatz von organischen oder anorganischen Farbstoffen beliebig eingefärbt werden. Die Farbstoffe können im weitesten Sinne auch als Füllstoff angesehen werden.

Ein besonderes Anwendungsgebiet der erfindungsgemäßen Polymere betrifft die Verwendung als kompostierbare Folie oder einer kompostierbaren Beschichtung als Außenlage von Windeln. Die Außenlage der Windeln verhindert wirksam den Durchtritt von Flüssigkeiten, die im Innern der Windel vom Fluff und Superabsorbern, bevorzugt von bioabbaubaren Superabsorbern, beispielsweise auf Basis von vernetzter Polyacrylsäure oder vernetztem Polyacrylamid, absorbiert werden. Als Innenlage der Windel kann man ein Faservlies aus einem Cellulosematerial verwenden. Die Außenlage der beschriebenen Windeln ist biologisch abbaubar und damit kompostierbar. Sie zerfällt beim Kompostieren, so daß die gesamte Windel verrottet, während mit einer Außenlage aus beispielsweise Polyethylen versehene Windeln nicht ohne vorherige Zerkleinerung oder aufwendige Abtrennung der Polyethylenfolie kompostiert werden können.

Eine weitere bevorzugte Verwendung der erfindungsgemäßen Polymere und Formmassen betrifft die Herstellung von Klebstoffen in an sich bekannter Weise (siehe beispielsweise Encycl. of Polym. Sc. and Eng. Vol.1, "Adhesive Compositions", S. 547 bis 577). Analog zur Lehre der EP-A 21 042 kann man die erfindungsgemäßen Polymere und Formmassen auch mit geeigneten klebrigmachenden thermoplastischen Harzen, bevorzugt Naturharzen, nach dort beschriebenen Methoden verarbeiten. Analog zur Lehre der DE-A 4,234,305 kann man die erfindungsgemäßen Polymere und Formmassen auch zu lösungsmittelfreien Klebstoffsystemen wie Hot-melt-Folien weiterverarbeiten.

Ein weiteres bevorzugtes Anwendungsgebiet betrifft die Herstellung vollständig abbaubarer Blends mit Stärkemischungen (bevorzugt mit thermoplastischer Stärke wie in der WO 90/05161 beschrieben) analog zu dem in der DE-A 42 37 535 beschriebenen Verfahren. Die erfindungsgemäßen Polymere und thermoplastischen Formmassen lassen sich nach bisherigen Beobachtungen auf Grund ihrer hydrophoben Natur, ihren mechanischen Eigenschaften, ihrer vollständigen Bioabbaubarkeit, ihrer guten Verträglichkeit mit thermoplastischer Stärke und nicht zuletzt wegen ihrer günstigen Rohstoffbasis vorteilhaft als synthetische Blendkomponente einsetzen.

Weitere Anwendungsgebiete betreffen beispielsweise die Verwendung der erfindungsgemäßen Polymere in landwirtschaftlichem Mulch, Verpackungsmaterial für Saatgut und Nährstoffe, Substrat in Klebefolien, Babyhöschen, Taschen, Bettücher, Flaschen, Kartons, Staubbeutel, Etiketten, Kissenbezüge, Schutzkleidung, Hygieneartikel, Taschentücher, Spielzeug und Wischer.

Eine weitere Verwendung der erfindungsgemäßen Polymere und Formmassen betrifft die Herstellung von Schäumen, wobei man im allgemeinen nach an sich bekannten Methoden vorgeht (siehe EP-A 372,846; Handbook of Polymeric foams and Foam Technology, Hanser Publisher, München, 1991, S. 375 bis 408). Üblicherweise wird dabei das erfindungsgemäße Polymere bzw. Formmasse zunächst aufgeschmolzen, gewünschtenfalls unter Zugabe von bis zu 5 Gew.-% Verbindung D, bevorzugt Pyromellitsäuredianhydrid und Trimellitsäureanhydrid, dann mit einem Treibmittel versetzt und die so erhaltene Mischung durch Extrusion vermindertem Druck ausgesetzt, wobei die Schäumung entsteht.

Die Vorteile der erfindungsgemäßen Polymere gegenüber bekannten bioabbaubaren Polymere liegen in einer günstigen Rohstoffbasis mit gut verfügbaren Ausgangsstoffen wie Adipinsäure, Terephthalsäure und gängigen Diolen, in interessanten mechanischen Eigenschaften durch Kombination von "harten" (durch die aromatischen Dicarbonsäuren wie beispielsweise Terephthalsäure) und "weichen" (durch die aliphatischen Dicarbonsäuren, wie beispielsweise Adipinsäure) Segmenten in der Polymerkette und der Variation der Anwendungen durch einfache Modifizierungen, in einem guten Abbauverhalten durch Mikroorganismen, besonders im Kompost und im Boden, und in einer gewissen Resistenz gegenüber Mikroorganismen in wäßrigen Systemen bei Raumtemperatur, was für viele Anwendungsbereiche besonders vorteilhaft ist. Durch den statistischen Einbau der aromatischen Dicarbonsäuren der Komponenten (a1) in verschiedenen Polymeren wird der biologische Angriff ermöglicht und damit die gewünschte biologische Abbaubarkeit erreicht.

Besonders vorteilhaft an den erfindungsgemäßen Polymere ist, daß durch maßgeschneiderte Rezepturen sowohl biologisches Abbauverhalten und mechanische Eigenschaften für den jeweiligen Anwendungszweck optimiert werden können.

Des weiteren können je nach Herstellverfahren vorteilhaft Polymere mit überwiegend statistisch verteilten Monomerbausteinen, Polymere mit überwiegend Blockstrukturen sowie Polymere mit überwiegend Blendstruktur oder Blends erhalten werden.

Beispiele

Enzym-Test

Die Polymere wurden in einer Mühle mit flüssigem Stickstoff oder Trockeneis gekühlt und fein gemahlen (je

größer die Oberfläche des Mahlguts, desto schneller der enzymatische Abbau). Zur eigentlichen Durchführung des Enzym-Tests wurden 30 mg fein gemahlenes Polymerpulver und 2 ml einer 20 mmol wäßrigen $K_2HPO_4/KH_2PO_4$-Pufferlösung (pH-Wert: 7,0) in ein Eppendorfreagenzgefäß (2 ml) gegeben und 3 h bei 37°C auf einem Schwenker equilibriert Anschließend wurden 100 units Lipase aus entweder Rhizopus arrhizus, Rhizopus delemar oder Pseudomonas pl. zugesetzt und 16 h bei 37°C unter Rühren (250 rpm) auf dem Schwenker inkubiert. Danach wurde die Reaktionsmischung durch eine Millipore®-Membran (0,45 μm) filtriert und der DOC (dissolved organic carbon) des Filtrats gemessen. Analog dazu wurden je eine DOC-Messung nur mit Puffer und Enzym (als Enzymkontrolle) und eine nur mit Puffer und Probe (als Blindwert) durchgeführt.

Die ermittelten ΔDOC-Werte (DOC(Probe + Enzym)-DOC(Enzymkontrolle)-DOC(Blindwert)) können als Maß für die enzymatische Abbaubarkeit der Proben angesehen werden. Sie sind jeweils im Vergleich zu einer Messung mit Pulver von Polycaprolacton® Tone P 787 (Union Carbide) dargestellt. Bei der Bewertung ist darauf zu achten, daß es sich nicht um absolut quantifizierbare Daten handelt. Auf den Zusammenhang zwischen Oberfläche des Mahlguts und Schnelligkeit des enzymatischen Abbaus wurde weiter oben bereits hingewiesen. Des weiteren können auch die Enzymaktivitäten schwanken.

Die Molekulargewichte wurden mittels Gelpermeationschromatographie (GPC) gemessen:

| | |
|---|---|
| stationäre Phase | 5 MIXED B-Polystyrolgelsäulen (7,5x300 mm, PL-gel 10 μ) der Fa. Polymer Laboratories; Temperierung: 35°C. |
| mobile Phase | Tetrahydrofuran (Fluß: 1,2 ml/min) |
| Eichung | Molgewicht 500-10000000 g/mol mit PS-Eichkit der Fa. Polymer Laboratories. |

Im Oligomerbereich Ethylbenzol/1,3-Diphenylbutan/1,3,5-Triphenylhexan/1,3,5,7-Tetraphenyloktan/1,3,5,7,9-Pentaphenyldekan

| | |
|---|---|
| Detektion: | RI (Brechungsindex) Waters 410<br>UV (bei 254 nm) Spectra Physics 100 |

Die Bestimmungen der Hydroxyl-Zahl (OH-Zahl) und Säure-Zahl (SZ) erfolgten nach folgenden Methoden:

(a) Bestimmung der scheinbaren Hydroxyl-Zahl

Zu ca. 1 bis 2 g exakt einqewogener Prüfsubßtanz wurden 10 ml Toluol und 9,8 ml Acetylierungsreagenz (s.u.) gegeben und 1 h bei 95°C unter Rühren erhitzt. Danach wurden 5 ml dest. Wasser zugeführt. Nach Abkühlen auf Raumtemperatur wurden 50 ml Tetrahydrofuran (THF) zugesetzt und mit ethanolischer KOH-Maßlösung gegen Wendepunkt potentiographisch titriert.

Der Versuch wurde ohne Prüfsubstanz wiederholt (Blindprobe).

Die scheinbare OH-Zahl wurde dann aufgrund folgender Formel ermittelt:

scheinb. OH-Zahl $c \cdot t \cdot 56{,}1 \cdot (V2-V1)/m$ (in mg KOH/g)

wobei c = Stoffmengenkonzentration der ethanol. KOH-Maßlösung in mol/l,

t = Titer der ethanol. KOH-Maßlösung

m = Einwaage in mg der Prüfsubstanz

V1 = Verbrauch der Maßlösung mit Prüfsubstanz in ml

V2 = Verbrauch der Maßlösung ohne Prügsubstanz in ml

bedeuten.

Verwendete Reagenzien:

ethanol. KOH-Maßlösung, c = 0,5 mol/l, Titer 0,9933
(Merck, Art.Nr. 1.09114)
Essigsäureanhydrid p.A. (Merck, Art.Nr. 42)
Pyridin p.A. (Riedel de Haen, Art.-Nr 33638)
Essigsäure p.A. (Merck, Art.Nr. 1.00063)
Acetylierungsreagenz: 810 ml Pyridin, 100 ml Essigsäureanhydrid und 9 ml Essigsäure
Wasser, deionisiert
THF und Toluol

(b) Bestimmung der Säurezahl (SZ)

Ca. 1 bis 1,5 g Prüfsubstanz wurden exakt eingewogen und mit 10 ml Toluol und 10 ml Pyridin versetzt und anschließend auf 95°C erhitzt. Nach dem Lösen wurde auf Raumtemperatur abgekühlt, 5 ml Wasser und 50 ml THF zugegeben und mit 0,1 N ethanol. KOHMaßlösung titriert.

Die Bestimmung wurde ohne Prüfsubstanz wiederholt (Blindprobe)

Die Säure-Zahl wurde dann aufgrund folgender Formel ermittelt:

$$SZ = c \cdot t \cdot 56{,}1 \cdot (V1-V2)/m \text{ (in mg KOH/g)}$$

wobei c = Stoffmengenkonzentration der ethanol. KOH-Maßlösung in mol/l,
t = Titer der ethanol. KOH-Maßlösung
m = Einwaage in mg der Prüfsubstanz
V1 = Verbrauch der Maßlösung mit Prüfsubstanz in ml
V2 = Verbrauch der Maßlösung ohne Prüfsubstanz in ml bedeuten.

Verwendete Reagenzien:

ethanol. KOH-Maßlösung, c = 0,1 mol/l, Titer = 0,9913
(Merck, Art.Nr. 9115)
Pyridin p.A. (Riedel de Haen, Art.Nr. 33638)
Wasser, deionisiert
THF und Toluol

(c) Bestimmung der OH-Zahl

Die OH-Zahl ergibt sich aus der Summe der scheinbaren OH-Zahl und der SZ:

OH-Zahl = scheinb. OH-Zah1 + SZ

Verwendete Abkürzungen:

| | |
|---|---|
| DOC | dissolved organic carbon |
| DMT | Dimethylterephthalat |
| PCL | Polycaprolacton® Tone P 787 (Union Carbide) |
| PMDA | Pyromellitsäuredianhydrid |
| SZ | Säurezahl |
| TBOT | Tetrabutylorthotitanat |
| VZ | Viskositätszahl (gemessen in o-Dichlorbenzol/Phenol (Gew.-Verhältnis 50/50) bei einer Konzentration von 0,5 Gew.-% Polymer bei einer Temperatur von 25°C |
| $T_m$ | "Schmelztemperatur" = Temperatur, bei der ein maximaler endothermer Wärmefluß auftritt (Extremum der DSC-Kurven) |
| $T_g$ | Glasübergangstemperatur (midpoint der DSC-Kurven) |

Die DSC-Messungen wurden mit einem DSC-Gerät 912+Thermal Analyzer 990 der Fa. DuPont durchgeführt. Die Temperatur- und Enthalpiekalibrierung erfolgte in üblicher Weise. Die Probeneinwaage betrug typischerweise 13 mg. Heiz- und Kühlraten betrugen - außer wenn anders vermerkt - 20 K/min. Die Proben wurden unter folgenden Bedingungen vermessen: 1. Aufheizender Lauf an Proben im Anlieferungszustand, 2. Schnelles Abkühlen aus der Schmelze, 3. Aufheizender Lauf an aus der Schmelze abgekühlten Proben (Proben aus 2). Die jeweils zweiten DSC-Läufe dienten dazu, nach Einprägen einer einheitlichen thermischen Vorgeschichte, einen Vergleich zwischen den verschiedenen Proben zu ermöglichen.

Beispiel 1 - Herstellung eines Polyetheresters P1 (nicht erfindungsgemäß)

(a) 4672 kg 1,4 Butandiol, 7000 kg Adipinsäure und 50 g Zinndioctoat wurden in einer Stickstoffatmosphäre bei einer Temperatur im Bereich von 230 bis 240°C zur Reaktion gebracht. Nach Abdestillieren der Hauptmenge des bei der Umsetzung gebildeten Wassers, wurden 10 g TBOT zur Reaktionsmischung gegeben. Nachdem die Säurezahl unter den Wert 1 gesunken war, wurde unter vermindertem Druck überschüssiges 1,4-Butandiol solange abdestilliert, bis eine OH-Zahl von 56 erreicht war.

(b) 2500 g des Polymers aus Bsp. 1(a), 5,6 kg DMT, 5,6 kg 1,4-Butandiol, 60 g PMDA, 6,22 kg Polyethylenglykol (mit Molekulargewicht 600 g/mol; Systol® T 122) und 20 g TBOT wurden in einen Rührkessel eingefüllt und unter Stickstoffatmosphäre unter langsamem Rühren auf 180°C erhitzt. Dabei wurde das während der Umesterungsreaktion gebildete Methanol abdestilliert. Innerhalb von 3 h wurde unter Erhöhung der Rührgeschwindigkeit auf 230°C erhitzt und nach einer weiteren Stunde noch 8 g 50 gew.-%ige wäßrige phosphorige Säure zugegeben. Innerhalb von 2 h wurde der Druck auf 5 mbar abgesenkt und bei 240°C noch 2,5 h bei < 2 mbar gehalten, wobei das im Überschuß eingesetzte Diol abdestillierte.

| | |
|---|---|
| OH-Zahl | 2 mg KOH/g |
| SZ | 4,5 mg KOH/g |
| VZ | 112 g/ml |
| $T_m$ | 108°C; Tg = -45°C (DSC, Anlieferungszustand) |

Beispiel 2 - Herstellung eines Polyetheresters Q2

Zu 300 g des Copolyetheresters aus Beispiel 1b wurden unter Rühren und unter Stickstoffatmosphäre bei 200°C tropfenweise innerhalb von 25 min 12,4 g des Bisoxalins Bis(2-ricinol-2-oxazolin)-tetramethylxyloldiurethan (Loxamid® VEP 8523 der Fa. Henkel, ein Bisoxazolin aus Ricinololoxazolin und 4,4'-Diphenylmethandiisocyanat, herstellbar gemäß DE-A 39 15 874) zugegeben, wobei die Schmelzviskosität zunahm und das Produkt sich bräunlich färbte.

| | |
|---|---|
| OH-Zahl | 3 mg KOH/g |
| SZ | 2 mg KOH/g |

Enzym-Test mit Rhizopus arrhizus: ΔDOC: 126 mg/l; zum Vergleich mit PCL: ΔDOC: 2588 mg/l.

Beispiel 3 - Herstellung eines weiteren Polyetheresters Q2

Zu 300 g des Copolyetheresters aus Beispiel 1b wurden unter Rühren und unter Stickstoffatmosphäre bei 200°C tropfenweise innerhalb von 25 min 3,5 g des Bisoxalins 1,4-Bis-(2-oxazolinyl)butan zugegeben, wobei die Schmelzviskosität zunahm und das Produkt sich bräunlich färbte.

| | |
|---|---|
| OH-Zahl | 3 mg KOH/g |
| SZ | 2 mg KOH/g |
| $T_m$ | 112°C; Tg = -42°C (DSC im Anlieferungszustand) |

Enzym-Test mit Rhizopus arrhizus: ΔDOC: 143 mg/l; zum Vergleich mit PCL: ΔDOC: 2588 mg/l.

## Patentansprüche

**1.** Biologisch abbaubare Polyetherester Q2 mit einem Molekulargewicht ($M_n$) im Bereich von 6000 bis 80000 g/mol, einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polyetherester Q2 bei einer Temperatur von 25°C), und einen Schmelzpunkt im Bereich von 50 bis 200°C, erhältlich durch Reaktion einer Mischung bestehend im wesentlichen aus

(d1) von 95 bis 99,9 Gew.-% Polyetherester P1, erhältlich durch Reaktion einer Mischung, bestehend im wesentlichen aus

(a1) einer Mischung, bestehend im wesentlichen aus
20 bis 95 mol-% Adipinsäure oder esterbildende Derivate davon oder Mischungen davon,
5 bis 80 mol-% Terephthalsäure oder esterbildende Derivate davon oder Mischungen davon, und
0 bis 5 mol-% einer sulfonatgruppenhaltigen Verbindung,

wobei die Summe der einzelnen Molprozentangaben 100 mol-% beträgt, und

(a2) einer Mischung aus Dihydroxyverbindungen, bestehend im wesentlichen aus

(a21) von 15 bis 99,8 mol-% einer Dihydroxyverbindung, ausgewählt aus der Gruppe bestehend aus $C_2$-$C_6$-Alkandiole und $C_5$-$C_{10}$-Cycloalkandiolen,

(a22) von 85 bis 0,2 mol-% einer Etherfunktionen-enthaltenden Dihydroxyverbindung gemäß Formel I

$$HO\text{-}[(CH_2)_n\text{-}O]_m\text{-}H \qquad I$$

in der n für 2, 3 oder 4 und m für eine ganze Zahl von 2 bis 250 stehen, oder Mischungen davon, wobei man das Molverhältnis von (a1) zu (a2) im Bereich von 0,4:1 bis 1,5:1 wählt, mit der Maßgabe, daß die Polyetherester P1 ein Molekulargewicht ($M_n$) im Bereich von 5000 bis 80000 g/mol, eine Viskositätszahl im Bereich von 30 bis 450 g/ml (gemessen in o-Dichlorbenzol/Phenol (Gew.-Verhältnis 50/50) bei einer Konzentration von 0,5 Gew.-% Polyetherester P1 bei einer Temperatur von 25°C) und einen Schmelzpunkt im Bereich von 50 bis 200°C aufweisen, und mit der weiteren Maßgabe, daß man von 0,01 bis 4 mol-%, bezogen auf die Molmenge der eingesetzten Komponente (a1), eine Verbindung D mit mindestens drei zur Esterbildung befähigten Gruppen zur Herstellung der Polyetherester P1 einsetzt, sowie der weiteren Maßgabe, daß die Polyetherester P1 sowohl Hydroxyl- als auch Carboxylendgruppen besitzen, wobei man das Molverhältnis von Carboxylendgruppen zu Hydroxylendgruppen größer als eins wählt,

(d2) von 0,1 bis 5 Gew.-% eines Bisoxazolins C1, wobei die Summe der einzelnen Molprozentangaben 100 mol-% beträgt, und

(d3) von 0 bis 5 mol-%, bezogen auf Komponente (a1) aus der Herstellung von P1, Verbindung D.

**2.** Biologisch abbaubare Polymere T1 mit einem Molekulargewicht ($M_n$) im Bereich von 10000 bis 100000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T1 bei einer Temperatur von 25 °C) und einem Schmelzpunkt im Bereich von 50 bis 235°C, erhältlich durch Umsetzung des Polyetheresters Q1 mit einem Molekulargewicht ($M_n$) im Bereich von 5000 bis 100000 g/mol, einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polyetherester Q1 bei einer Temperatur von 25°C), und einen Schmelzpunkt im Bereich von 50 bis 235°C, mit sowohl Hydroxyl- als auch Carboxylendgruppen, wobei man das Molverhältnis von Carboxylendgruppen zu Hydroxylendgruppen größer als eins wählt, erhältlich durch Reaktion einer Mischung bestehend im wesentlichen aus

(c1) Polyetherester P1,

(c2) 0,01 bis 50 Gew.-%, bezogen auf (c1), Hydroxycarbonsäure B1, und

(c3) 0 bis 5 mol-%, bezogen auf Komponente (a1) aus der Herstellung von P1, Verbindung D
mit (e1) 0,1 bis 5 Gew.-%, bezogen auf den Polyetherester Q1, Bisoxazolin C1 sowie mit (e2) 0 bis 5 mol-%, bezogen auf Komponente (a1) aus der Herstellung von Polyetherester Q1 über den Polyetherester P1, Verbindung D.

**3.** Biologisch abbaubare Polymere T2 mit einem Molekulargewicht ($M_n$) im Bereich von 10000 bis 100000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T2 bei einer Temperatur von 25°C) und einem Schmelzpunkt im Bereich von 50 bis 235°C, erhältlich durch Umsetzung des Polyetheresters Q2 mit

(f1) 0,01 bis 50 Gew.-%, bezogen auf Polyetherester Q2, Hydroxycarbonsäure B1 sowie mit

(f2) 0 bis 5 mol-%, bezogen auf Komponente (a1) aus der Herstellung von Polyetherester Q2 über den Polyetherester P1, Verbindung D.

**4.** Biologisch abbaubare Polymere T3 mit einem Molekulargewicht ($M_n$) im Bereich von 10000 bis 100000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T3 bei einer Temperatur von 25°C) und einem Schmelzpunkt im Bereich von 50 bis 235°C, erhältlich durch Umsetzung von

(g1) Polyetherester P2 erhältlich durch Reaktion einer Mischung, bestehend im wesentlichen aus

(b1) einer Mischung, bestehend im wesentlichen aus
20 bis 95 mol-% Adipinsäure oder esterbildende Derivate davon oder Mischungen davon,
5 bis 80 mol-% Terephthalsäure oder esterbildende Derivate davon oder Mischungen davon, und
0 bis 5 mol-% einer sulfonatgruppenhaltigen Verbindung, wobei die Summe der einzelnen Molprozentangaben 100 mol-% beträgt,

(b2) einer Mischung aus Dihydroxyverbindungen ($a_2$),
wobei man das Molverhältnis von (b1) zu (b2) im Bereich von 0,4:1 bis 1,25:1 wählt,

(b3) von 0,01 bis 100 Gew.-%, bezogen auf Komponente (b1) einer Hydroxycarbonsäure B1, und

(b4) von 0,01 bis 3,5 mol-%, bezogen auf Komponente (b1) Verbindung D,
wobei die Hydroxycarbonsäure B1 definiert ist durch die Formeln IIa oder IIb

$$HO-[-C(O)-G-O-]_pH$$

IIa

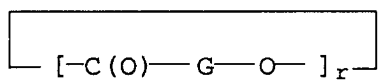

IIb

in der p eine ganze Zahl von 1 bis 1500 und r eine ganze Zahl von 1 bis 4 bedeuten, und G für einen Rest steht, der ausgewählt ist aus der Gruppe bestehend aus Phenylen, $-(CH_2)_k-$, wobei k eine ganze Zahl von 1 bis 5 bedeutet, -C(R)H- und-$C(R)HCH_2$, wobei R für Methyl oder Ethyl steht,
wobei die Polyetherester P2 ein Molekulargewicht ($M_n$ im Bereich von 5000 bis 80000 g/mol, eine Viskositätszahl im Bereich von 30 bis 450 g/ml (gemessen in o-Dichlorbenzol/Phenol (Gew.-Verhältnis 50/50) bei einer Konzentration von 0,5 Gew.-% Polyetherester P2 bei einer Temperatur von 25°C) und einen Schmelzpunkt im Bereich von 50 bis 235°C aufweisen, und sowohl Hydroxyl- als auch Carboxylendgruppen besitzen, wobei man das Molverhältnis von Carboxylendgruppen zu Hydroxylendgruppen größer als eins wählt,
oder (g2) einer Mischung bestehend im wesentlichen aus Polyetherester P1 und 0,01 bis 50 Gew.-%, bezogen auf Polyetherester P1, Hydroxycarbonsäure B1, oder (g3) einer Mischung bestehend im wesentlichen aus Polyetherestern P1, die eine unterschiedliche Zusammensetzung voneinander aufweisen,
mit 0,1 bis 5 Gew.-%, bezogen auf die Menge der eingesetzten Polyetherester, Bisoxazolin C1 sowie
mit 0 bis 5 mol-%, bezogen auf die jeweiligen Molmengen an Komponente (a1), die zur Herstellung der eingesetzten Polyetherester (g1) bis (g3) eingesetzt wurden, Verbindung D.

**5.** Biologisch abbaubare thermoplastische Formmassen T4, erhältlich durch Mischen in an sich bekannter Weise von

(h1) 99,5 bis 0,5 Gew.-% Polyetherester Q2 gemäß Anspruch 1 mit

(h2) 0,5 bis 99,5 Gew.-% Hydroxycarbonsäure B1.

**6.** Verfahren zur Herstellung der biologisch abbaubaren Polyetherester Q2 gemäß Anspruch 1 in an sich bekannter Weise, **dadurch gekennzeichnet, daß** man eine Mischung, bestehend im wesentlichen aus

(d1) von 95 bis 99,9 Gew.-% Polyetherester P1,

(d2) von 0,1 bis 5 Gew.-% eines Bisoxazolins C1 und

(d3) von 0 bis 5 mol-%, bezogen auf Komponente (a1) aus der Herstellung von P1, Verbindung D

zur Reaktion bringt.

**7.** Verfahren zur Herstellung der biologisch abbaubaren Polymeren T1 gemäß Anspruch 2 in an sich bekannter Weise, **dadurch gekennzeichnet, daß** man Polyetheresters Q1 gemäß Anspruch 2 mit (e1) 0,1 bis 5 gew.-%, bezogen auf den Polyetherester Q1, Bisoxazolin C1 sowie mit (e2) 0 bis 5 mol-%, bezogen auf Komponente (a1) aus der Herstellung von P1 sowie Polyetherester Q1, Verbindung D zur Reaktion bringt.

**8.** Verfahren zur Herstellung der biologisch abbaubaren Polymeren T2 gemäß Anspruch 3 in an sich bekannter Weise, **dadurch gekennzeichnet, daß** man Polyetherester Q2 mit

(f1) 0,01 bis 50 Gew.-%, bezogen auf Polyetherester Q2, Hydroxycarbonsäure B1 sowie mit

(f2) 0 bis 5 mol-%, bezogen auf Komponente (a1) aus der Herstellung von P1 sowie des Polyetheresters Q2, Verbindung D,

zur Reaktion bringt.

**9.** Verfahren zur Herstellung der biologisch abbaubaren Polymeren T3 gemäß Anspruch 4 in an sich bekannter Weise, **dadurch gekennzeichnet, daß** man

(g1) Polyetherester P2, oder

(g2) eine Mischung, bestehend im wesentlichen aus Polyetherester P1 und 0,01 bis 50 Gew.-%, bezogen auf Polyetherester P1, Hydroxycarbonsäure B1, oder

(g3) eine Mischung, bestehend im wesentlichen aus Polyetherestern P1, die eine unterschiedliche Zusammensetzung voneinander aufweisen,

mit 0,1 bis 5 Gew.-%, bezogen auf die Menge der eingesetzten Polyetherester, Bisoxazolin C1 sowie
mit 0 bis 5 mol-%, bezogen auf die jeweiligen Molmengen an Komponente (a1), die zur Herstellung der eingesetzten Polyetherester (g1) bis (g3) eingesetzt wurden, Verbindung D, zur Reaktion bringt.

**10.** Verfahren zur Herstellung der biologisch abbaubaren thermoplastischen Formmassen T4 gemäß Anspruch 5 in an sich bekannter Weise, **dadurch gekennzeichnet, daß** man
99,5 bis 0,5 Gew.-% Polyetherester P1 gemäß Anspruch 1 oder Polyetherester Q2 gemäß Anspruch 1 mit
0,5 bis 99,5 Gew.-% Hydroxycarbonsäure B1 mischt.

**11.** Verwendung der biologisch abbaubaren Polymere gemäß den Ansprüchen 1 bis 4 oder der thermoplastischen Formmassen gemäß Anspruch 5 oder hergestellt gemäß den Ansprüchen 6 bis 10 zur Herstellung von kompostierbaren Formkörpern.

**12.** Verwendung der biologisch abbaubaren Polymere gemäß den Ansprüchen 1 bis 4 oder der thermoplastischen Formmassen gemäß Anspruch 5 oder hergestellt gemäß den Ansprüchen 6 bis 10 zur Herstellung von Klebstoffen.

**13.** Kompostierbare Formkörper, erhältlich durch die Verwendung gemäß Anspruch 11.

**14.** Klebstoffe, erhältlich durch die Verwendung gemäß Anspruch 12.

**15.** Verwendung der biologisch abbaubaren Polymere gemäß den Ansprüchen 1 bis 4 oder der thermoplastischen Formmassen gemäß Anspruch 5 oder hergestellt gemäß den Ansprüchen 6 bis 10 zur Herstellung von biologisch abbaubaren Blends, enthaltend im wesentlichen die erfindungsgemäßen Polymere und Stärke.

**16.** Biologisch abbaubare Blends, erhältlich durch die Verwendung gemäß Anspruch 15.

**17.** Verfahren zur Herstellung biologisch abbaubarer Blends gemäß Anspruch 16 in an sich bekannter Weise, **dadurch gekennzeichnet, daß** man Stärke mit den erfindungsgemäßen Polymeren mischt.

**18.** Verwendung der biologisch abbaubaren Polymere gemäß den Ansprüchen 1 bis 4 oder der thermoplastischen Formmassen gemäß Anspruch 5 oder hergestellt gemäß den Ansprüchen 6 bis 10 zur Herstellung von biologisch abbaubaren Schäumen.

**19.** Biologisch abbaubare Schäume, erhältlich durch die Verwendung gemäß Anspruch 18.

**20.** Biologisch abbaubare Papierbeschichtungsmittel, erhältlich durch die Verwendung von biologisch abbaubaren Polymeren gemäß den Ansprüchen 1 bis 4 oder der thermoplastischen Formmassen gemäß Anspruch 5 oder hergestellt gemäß den Ansprüchen 6 bis 10.

## Claims

**1.** A biodegradable polyether ester Q2 with a molecular weight ($M_n$) in the range from 6000 to 80,000 g/mol, a viscosity number in the range from 30 to 450 g/ml (measured in o-dichlorobenzene/phenol (50/50 ratio by weight) at a concentration of 0.5 % by weight of polyether ester Q2 at 25°C) and a melting point in the range from 50 to 200°C, obtainable by reacting a mixture essentially comprising

(d1) from 95 to 99.9 % by weight of polyether ester P1, obtainable by reacting a mixture essentially comprising

(a1) a mixture essentially comprising
20-95 mol% of adipic acid or ester-forming derivatives thereof or mixtures thereof,
5-80 mol% of terephthalic acid or ester-forming derivatives thereof or mixtures thereof, and
0-5 mol% of a compound containing sulfonate groups,
where the total of the individual mole percentages is 100 mol%, and

(a2) a mixture of dihydroxy compounds essentially comprising

(a21) from 15 to 99.8 mol% of a dihydroxy compound selected from the group consisting of $C_2$-$C_6$-alkanediols and $C_5$-$C_{10}$-cycloalkanediols,

(a22) from 85 to 0.2 mol% of a dihydroxy compound containing ether functionalities of the formula I

$$HO\text{-}[(CH_2)_n\text{-}O]_m\text{-}H \qquad \text{I}$$

where n is 2, 3 or 4 and m is an integer from 2 to 250, or mixtures thereof,
where the molar ratio of (a1) to (a2) is chosen in the range from 0.4:1 to 1.5:1, with the proviso that the polyether ester P1 has a molecular weight ($M_n$) in the range from 5000 to 80,000 g/mol, a viscosity number in the range from 30 to 450 g/ml (measured in o-dichlorobenzene/phenol (50/50 ratio by weight) at a concentration of 0.5 % by weight of polyether ester P1 at 25°C) and a melting point in the range from 50 to 200°C, and with the further proviso that from 0.01 to 4 mol%, based on the molar quantity of component (a1) employed, of a compound D with at least three groups capable of ester formation are employed to prepare the polyether ester P1, and the further proviso that the polyether ester P1 has both hydroxyl and carboxyl end groups, with the molar ratio of carboxyl end groups to hydroxyl end groups being chosen to be greater than one

(d2) from 0.1 to 5 % by weight of a bisoxazoline C1, where the total of the individual mole percentages is 100 mol%, and

(d3) from 0 to 5 mol%, based on component (a1) from the preparation of P1, of compound D.

**2.** A biodegradable polymer T1 with a molecular weight ($M_n$) in the range from 10,000 to 100,000 g/mol, with a viscosity number in the range from 30 to 450 g/ml (measured in o-dichlorobenzene/phenol (50/50 ratio by weight) at a concentration of 0.5 % by weight of polymer T1 at 25°C) and a melting point in the range from 50 to 235°C, obtainable by reacting the polyether ester Q1 with a molecular weight ($M_n$) in the range from 5000 to 100,000 g/mol, a viscosity number in the range from 30 to 450 g/ml (measured in o-dichlorobenzene/phenol (50/50 ratio by weight) at a concentration of 0.5 % by weight of polyether ester Q1 at 25°C) and a melting point in the range from

50 to 235°C, with both hydroxyl and carboxyl end groups, with the molar ratio of carboxyl end groups to hydroxyl end groups being chosen to be greater than one, obtainable by reacting a mixture essentially comprising

(c1) polyether ester P1,

(c2) 0.01-50 % by weight, based on (c1), of hydroxy carboxylic acid B1 and

(c3) 0-5 mol%, based on component (a1) from the preparation of P1, of compound D.

with (e1) 0.1-5 % by weight, based on the polyether ester Q1, of bisoxazoline C1 and with (e2) 0-5 mol%, based on component (a1) from the preparation of polyether ester Q1 via the polyether ester P1, of compound D.

**3.** A biodegradable polymer T2 with a molecular weight ($M_n$) in the range from 10,000 to 100,000 g/mol, with a viscosity number in the range from 30 to 450 g/ml (measured in o-dichlorobenzene/phenol (50/50 ratio by weight) at a concentration of 0.5 % by weight of polymer T2 at 25°C) and a melting point in the range from 50 to 235°C, obtainable by reacting the polyether ester Q2 with

(f1) 0.01-50 % by weight, based on the polyether ester Q2, of hydroxy carboxylic acid B1 and with

(f2) 0-5 mol%, based on component (a1) from the preparation of polyether ester Q2 via the polyether ester P1, of compound D.

**4.** A biodegradable polymer T3 with a molecular weight ($M_n$) in the range from 10,000 to 100,000 g/mol, with a viscosity number in the range from 30 to 450 g/ml (measured in o-dichlorobenzene/phenol (50/50 ratio by weight) at a concentration of 0.5 % by weight of polymer T3 at 25°C) and a melting point in the range from 50 to 235°C, obtainable by reacting

(g1) polyether ester P2 obtainable by reacting a mixture essentially comprising

(b1) a mixture essentially comprising
20-95 mol% of adipic acid or ester-forming derivatives thereof or mixtures thereof,
5-80 mol% of terephthalic acid or ester-forming derivatives thereof or mixtures thereof, and
0-5 mol% of a compound containing sulfonate groups,
where the total of the individual mole percentages is 100 mol%,

(b2) a mixture of dihydroxy compounds (a2),
where the molar ratio of (b1) to (b2) is chosen in the range from 0.4:1 to 1.25:1,

(b3) from 0.01 to 100 % by weight, based on component (b1), of a hydroxy carboxylic acid B1, and

(b4) from 0.01 to 3.5 mol%, based on component (b1), of compound D,

where the hydroxy carboxylic acid B1 is defined by the formulae IIa or IIb

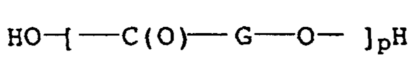

IIa

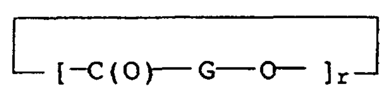

IIb

where p is an integer from 1 to 1500 and r is an integer from 1 to 4, and G is a radical which is selected from the group consisting of phenylene, $-(CH_2)_k-$, where k is an integer from 1 to 5, -C(R)H- and $-C(R)HCH_2$, where R is methyl or ethyl,
where the polyether ester P2 has a molecular weight ($M_n$) in the range from 5000 to 80,000 g/mol, a viscosity number in the range from 30 to 450 g/ml (measured in o-dichlorobenzene/phenol (50/50 ratio by weight) at a concentration of 0.5 % by weight of polyether ester P2 at 25°C) and a melting point in the range from 50 to 235°C,

and has both hydroxyl and carboxyl end groups, with the molar ratio of carboxyl end groups to hydroxyl end groups being chosen to be greater than one,

or (g2) a mixture essentially comprising polyether ester P1 and 0.01-50 % by weight, based on polyether ester P1, of hydroxy carboxylic acid B1, or (g3) a mixture essentially comprising polyether esters P1 which differ from one another in composition,
with 0.1-5 % by weight, based on the quantity of the polyether esters employed, of bisoxazoline C1 and with 0-5 mol%, based on the respective molar quantities of component (a1) employed to prepare the polyether esters (g1) to (g3) employed, of compound D.

**5.** A biodegradable thermoplastic molding composition T4 obtainable by mixing in a conventional way

(h1) 99.5-0.5 % by weight of polyether ester Q2 as claimed in claim 1 with

(h2) 0.5-99.5 % by weight of hydroxy carboxylic acid B1.

**6.** A process for preparing a biodegradable polyether ester Q2 as claimed in claim 1 in a conventional way, which comprises reacting a mixture essentially comprising

(d1) from 95 to 99.9 % by weight of polyether ester P1,

(d2) from 0.1 to 5 % by weight of a bisoxazoline C1 and

(d3) from 0 to 5 mol%, based on component (a1) from the preparation of P1, of compound D.

**7.** A process for preparing a biodegradable polymer T1 as claimed in claim 2 in a conventional way, which comprises reacting a polyether ester Q1 as claimed in claim 2 with (e1) 0.1-5 % by weight, based on the polyether ester Q1, of bisoxazoline C1 and with (e2) 0-5 mol%, based on component (a1) from the preparation of P1 and polyether ester Q1, of compound D.

**8.** A process for preparing a biodegradable polymer T2 as claimed in claim 3 in a conventional way, which comprises reacting a polyether ester Q2 with

(f1) 0.01-50 % by weight, based on the polyether ester Q2, of hydroxy carboxylic acid B1 and with

(f2) 0-5 mol%, based on component (a1) from the preparation of P1 and of the polyether ester Q2, of compound D.

**9.** A process for preparing a biodegradable polymer T3 as claimed in claim 4 in a conventional way, which comprises reacting

(g1) polyether ester P2, or

(g2) a mixture essentially comprising polyether ester P1 and 0.01-50 % by weight, based on polyether ester P1, of hydroxy carboxylic acid B1, or

(g3) a mixture essentially comprising polyether esters P1 which differ from one another in composition,

with 0.1-5 % by weight, based on the quantity of the polyether esters employed, of bisoxazoline C1 and
with 0-5 mol%, based on the respective molar quantities of component (a1) employed to prepare the polyether esters (g1) to (g3) employed, of compound D.

**10.** A process for preparing a biodegradable thermoplastic molding composition T4 as claimed in claim 5 in a conventional way, which comprises mixing
99.5-0.5 % by weight of polyether ester P1 as claimed in claim 1 or polyether ester Q2 as claimed in claim 1 with 0.5-99.5 % by weight of hydroxy carboxylic acid B1.

**11.** The use of the biodegradable polymers as claimed in claims 1 to 4 or of the thermoplastic molding compositions

as claimed in claim 5 or prepared as claimed in claims 6 to 10 for the production of compostable moldings.

**12.** The use of the biodegradable polymers as claimed in claims 1 to 4 or of the thermoplastic molding compositions as claimed in claim 5 or prepared as claimed in claims 6 to 10 for the production of adhesives.

**13.** A compostable molding obtainable by the use as claimed in claim 11.

**14.** An adhesive obtainable by the use as claimed in claim 12.

**15.** The use of the biodegradable polymers as claimed in claims 1 to 4 or of the thermoplastic molding compositions as claimed in claim 5 or prepared as claimed in claims 6 to 10 for the production of biodegradable blends essentially comprising the polymers according to the invention and starch.

**16.** A biodegradable blend obtainable by the use as claimed in claim 15.

**17.** A process for producing a biodegradable blend as claimed in claim 16 in a conventional way, which comprises mixing starch with the polymers according to the invention.

**18.** The use of the biodegradable polymers as claimed in claims 1 to 4 or of the thermoplastic molding compositions as claimed in claim 5 or prepared as claimed in claims 6 to 10 for the production of biodegradable foams.

**19.** A biodegradable foam obtainable by the use as claimed in claim 18.

**20.** A biodegradable paper coating agent obtainable by the use of biodegradable polymers as claimed in claims 1 to 4 or of the thermoplastic molding compositions as claimed in claim 5 or prepared as claimed in claims 6 to 10.

## Revendications

**1.** Polyétheresters biologiquement dégradables Q2, d'un poids moléculaire ($M_n$) qui varie de 6000 à 80000 g/mole, d'un indice de viscosité qui varie de 30 à 450 g/ml (mesuré dans un mélange de o-dichlorobenzène et de phénol (rapport pondéral 50/50) à une concentration de 0,5% en poids de polyétherester Q2 et à la température de 25°C) et d'un point de fusion qui varie de 50 à 200°C, que l'on peut obtenir par la réaction d'un mélange essentiellement constitué de

(d1) 95 à 99,9% en poids de polyétheresters P1, que l'on peut obtenir par réaction d'un mélange, essentiellement constitué de

(a1) un mélange essentiellement constitué de
20 à 95% molaires d'acide adipique ou de dérivés estérogènes de celui-ci ou de leurs mélanges,
5 à 80% molaires d'acide téréphtalique ou de dérivés estérogènes de celui-ci ou de leurs mélanges et
0 à 5% molaires d'un composé contenant des radicaux sulfonate,
où la somme des indications en pourcentages molaires individuelles totalise 100% molaires et

(a2) un mélange de composés dihydroxylés, essentiellement constitué de

(a21) 15 à 99,8% molaires d'un composé dihydroxylé, choisi dans le groupe constitué par des alcanediols en $C_2$ à $C_6$ et des cycloalcanediols en $C_5$ à $C_{10}$,

(a22) 85 à 0,2% molaires d'un composé dihydroxylé contenant des fonctions éther répondant à la formule I :

$$HO-[(CH_2)_n-O]_m-H \qquad I$$

dans laquelle n est égal à 2, 3 ou 4 et m représente un nombre entier dont la valeur varie de 2 à 250 ou de leurs mélanges,
où l'on choisit le rapport molaire de (a1) à (a2) dans la plage de 0,4:1 à 1,5:1, avec la condition que les polyétheresters P1 présentent un poids moléculaire ($M_n$) qui varie dans la plage de 5000 à 80000 g/mole, un indice de viscosité qui varie de 30 à 450 g/ml (mesuré dans un mélange de o-dichlorobenzène et de phénol (rapport pondéral 50/50) à une concentration de 0,5% en poids de polyétherester P1, à la température de 25°C) et un point de fusion qui fluctue dans la plage de 50 à 200°C et avec la condition supplémentaire que l'on utilise 0,01 à 4% molaires, par rapport à la quantité molaire du composant (a1) mis en oeuvre, d'un composé D comportant au moins trois radicaux estérogènes pour la préparation des polyétheresters P1, ainsi qu'avec la condition supplémentaire que les polyétheresters P1 possèdent des radicaux terminaux, aussi bien hydroxyle qu'également carboxyle, où l'on choisit un rapport molaire des radicaux terminaux carboxyle aux radicaux terminaux hydroxyle supérieur à un,

(d2) 0,1 à 5% en poids d'une bisoxazoline C1, où la somme des indications en pourcentages molaires individuelles totalise 100% molaires et
(d3) 0 à 5% molaires, par rapport au composant (a1) de la préparation de P1, d'un composé D.

**2.** Polymères biologiquement dégradables T1 d'un poids moléculaire ($M_n$) qui varie de 10000 à 100000 g/mole, d'un indice de viscosité qui varie de 30 à 450 g/ml (mesuré dans un mélange de o-dichlorobenzène et de phénol (rapport pondéral 50/50) à la concentration de 0,5% en poids de polymère T1 et à la température de 25°C) et d'un point de fusion qui varie de 50 à 235°C, que l'on peut obtenir par la réaction du polyétherester Q1 d'un poids moléculaire ($M_n$) qui varie de 5000 à 100000 g/mole, d'un indice de viscosité qui varie de 30 à 450 g/ml (mesuré dans un mélange de o-dichlorobenzène et de phénol (rapport pondéral 50/50) à la concentration de 0,5% en poids de polyétherester Q1 et à la température de 25°C) et d'un point de fusion qui varie de 50 à 235°C avec des radicaux terminaux, aussi bien hydroxyle qu'également carboxyle, où l'on choisit un rapport molaire des radicaux terminaux carboxyle aux radicaux terminaux hydroxyle supérieur à un, que l'on peut obtenir par la réaction d'un mélange essentiellement constitué de

(c1) polyétheresters P1,
(c2) 0,01 à 50% en poids, par rapport à (c1), d'acide hydroxycarboxylique B1 et
(c3) 0 à 5% molaires, par rapport au composant (a1) de la préparation de P1, d'un composé D,
avec (e1) 0,1 à 5% en poids, par rapport au polyétherester Q1, de bisoxazoline C1, ainsi qu'avec (e2) 0 à 5% molaires, par rapport au composant (a1) de la préparation de polyétheresters Q1 en passant par les polyétheresters P1. d'un composé D.

**3.** Polymères biologiquement dégradables T2 d'un poids moléculaire ($M_n$) qui varie de 10000 à 100000 g/mole, d'un indice de viscosité qui varie de 30 à 450 g/ml (mesuré dans un mélange de o-dichlorobenzène et de phénol (rapport pondéral 50/50) à la concentration de 0,5% en poids de polymère T2 et à la température de 25°C) et d'un point de fusion qui varie de 50 à 235°C, que l'on peut obtenir par la réaction du polyétherester Q2 avec

(f1) 0,01 à 50% en poids, par rapport aux polyétheresters Q2, d'acide hydroxycarboxylique B1, ainsi qu'avec
(f2) 0 à 5% molaires, par rapport au composant (a1) de la préparation de polyétheresters Q2 en passant par les polyétheresters P1, d'un composé D.

**4.** Polymères biologiquement dégradables T3, d'un poids moléculaire ($M_n$) qui varie de 10000 à 100000 g/mole, d'un indice de viscosité qui varie de 30 à 450 g/ml (mesuré dans un mélange de o-dichlorobenzène et de phénol (rapport pondéral 50/50) à la concentration de 0,5% en poids de polymère T3 et à la température de 25°C) et d'un point de fusion qui varie de 50 à 235°C, que l'on peut obtenir par la réaction

(g1) de polyétheresters P2, que l'on peut obtenir par la réaction d'un mélange essentiellement constitué de
(b1) un mélange, essentiellement constitué de
20 à 95% molaires d'acide adipique ou de dérivés estérogènes de celui-ci, ou de leurs mélanges,
5 à 80% molaires d'acide téréphtalique, ou de dérivés estérogènes de celui-ci, ou de leurs mélanges et
0 à 5% molaires d'un composé contenant des radicaux sulfonate, où la somme des indications en pourcentages molaires individuelles totalise 100% molaires,
(b2) un mélange de composés dihydroxylés (a2),
où l'on choisit le rapport molaire de (b1) à (b2) dans la plage de 0,4:1 à 1,25:1,
(b3) 0,01 à 100% en poids, par rapport au composant (b1), d'un acide hydroxycarboxylique B1 et
(b4) 0,01 à 3,5% molaires, par rapport au composant (b1), d'un composé D,

où l'acide hydroxycarboxylique B1 est défini par les formules IIa ou IIb :

$$HO-[-C(O)-G-O-]_pH$$

IIa

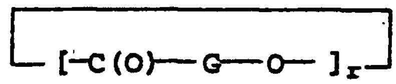

IIb

dans lesquelles p représente un nombre entier dont la valeur varie de 1 à 1500 et r représente un nombre entier dont la valeur varie de 1 à 4 et G représente un reste choisi dans le groupe constitué des radicaux phénylène, $-(CH_2)_k-$, où k représente un nombre entier dont la valeur varie de 1 à 5, -C(R)H- et $-C(R)HCH_2$ où R représente le radical méthyle ou éthyle,

où les polyétheresters P2 présentent un poids moléculaire ($M_n$) qui varie de 5000 à 80000 g/mole, un indice de viscosité qui varie de 30 à 450 g/ml (mesuré dans un mélange de o-dichlorobenzène et de phénol (rapport pondéral 50/50) à la concentration de 0,5% en poids de polyétherester P2 et à la température de 25°C) et un point de fusion qui fluctue de 50 à 235°C et possèdent des radicaux terminaux, aussi bien hydroxyle qu'également carboxyle, où l'on choisit un rapport molaire des radicaux terminaux carboxyle aux radicaux terminaux hydroxyle supérieur à un,

ou (g2) d'un mélange constitué essentiellement de polyétheresters P1 et de 0,01 à 50% en poids, par rapport aux polyétheresters P1, d'acide hydroxycarboxylique B1, ou (g3) d'un mélange essentiellement constitué de polyétheresters P1, qui présentent une composition qui diffère les uns des autres,

avec 0,1 à 5% en poids, par rapport à la quantité des polyétheresters mis en oeuvre, de bisoxazoline C1, ainsi que

avec 0 à 5% molaires, par rapport aux proportions molaires concernées en composant (a1), qui furent mises en oeuvre pour la préparation des polyétheresters mis en oeuvre (g1) à (g3), d'un composé D.

**5.** Masses à mouler thermoplastiques et biologiquement dégradables T4, que l'on peut obtenir par le mélange, d'une manière en soi connue, de

(h1) 99,5 à 0,5% en poids de polyétheresters Q2 suivant la revendica tion 1 avec
(h2) 0,5 à 99,5% en poids d'acide hydroxycarboxylique B1.

**6.** Procédé de préparation de polyétheresters biologiquement dégradables Q2 suivant la revendication 1, d'une manière en soi connue, **caractérisé en ce que** l'on fait réagir un mélange essentiellement constitué de

(d1) 95 à 99,9% en poids de polyétheresters P1,
(d2) 0,1 à 5% en poids d'une bisoxazoline C1 et
(d3) 0 à 5% molaires, par rapport au composant (a1) de la préparation de P1, d'un composé D.

**7.** Procédé de préparation de polymères biologiquement dégradables T1 suivant la revendication 2, d'une manière en soi connue, **caractérisé en ce que** l'on fait réagir des polyétheresters Q1 suivant la revendication 2 avec (e1) 0,1 à 5% en poids, par rapport aux polyétheresters Q1, de bisoxazoline C1, ainsi qu'avec (e2) 0 à 5% molaires, par rapport au composant (a1) de la préparation de P1, comme aussi de polyétheresters Q1, d'un composé D.

**8.** Procédé de préparation de polymères biologiquement dégradables T2 suivant la revendication 3, d'une manière en soi connue, **caractérisé en ce que** l'on fait réagir des polyétheresters Q2 avec

(f1) 0,01 à 50% en poids, par rapport au polyétheresters Q2, d'acide hydroxycarboxylique B1, ainsi qu'avec
(f2) 0 à 5% molaires, par rapport au composant (a1) de la préparation de P1, comme aussi des polyétheresters Q2, d'un composé D.

**9.** Procédé de préparation de polymères biologiquement dégradables T3 suivant la revendication 4, d'une manière en soi connue, **caractérisé en ce que** l'on fait réagir

(g1) des polyétheresters P2, ou
(g2) un mélange, essentiellement constitué de polyétheresters P1 et de 0,01 à 50% en poids, par rapport au polyétheresters P1, d'acide hydroxycarboxylique B1, ou
(g3) un mélange, essentiellement constitué de polyétheresters P1 qui présentent une composition différente les uns des autres,

avec 0,1 à 5% en poids, par rapport à la proportion des polyétheresters mis en oeuvre, de bisoxazoline C1, ainsi que avec 0 à 5% molaires, par rapport aux proportions molaires concernées en composant (a1), qui furent mises en oeuvre pour la préparation des polyétheresters (g1) à (g3) mis en oeuvre, d'un composé D.

**10.** Procédé de préparation de masses à mouler thermoplastiques et biologiquement dégradables T4 suivant la revendication 5, d'une manière en soi connue, **caractérisé en ce que** l'on mélange

99,5 à 0,5% en poids de polyétheresters P1 suivant la revendication 1 ou de polyétheresters Q2 suivant la revendication 1 avec 0,5 à 99,5% en poids d'acide hydroxycarboxylique B1.

**11.** Utilisation des polymères biologiquement dégradables suivant l'une quelconque des revendications 1 à 4 ou des masses à mouler thermoplastiques suivant la revendication 5 ou que l'on obtient suivant l'une quelconque des revendications 6 à 10, en vue de la fabrication d'articles moulés compostables.

**12.** Utilisation des polymères biologiquement dégradables suivant l'une quelconque des revendications 1 à 4 ou des masses à mouler thermoplastiques suivant la revendication 5, ou que l'on obtient suivant l'une quelconque des revendications 6 à 10, pour la fabrication de colles.

**13.** Articles moulés compostables, que l'on peut obtenir par l'utilisation suivant la revendication 11.

**14.** Colles, que l'on peut obtenir par l'utilisation suivant la revendication 17.

**15.** Utilisation des polymères biologiquement dégradables suivant l'une quelconque des revendications 1 à 4 ou des masses à mouler thermoplastiques suivant la revendication 5, ou que l'on obtient suivant l'une quelconque des revendications 6 à 10, pour la préparation de mélanges biologiquement dégradables, contenant essentiellement les polymères conformes à l'invention et de l'amidon.

**16.** Mélanges biologiquement dégradables, que l'on peut obtenir par l'utilisation suivant la revendication 15.

**17.** Procédé de préparation de mélanges biologiquement dégradables suivant la revendication 16, de manière en soi connue, **caractérisé en ce que** l'on mélange de l'amidon aux polymères conformes à l'invention.

**18.** Utilisation des polymères biologiquement dégradables suivant l'une quelconque des revendications 1 à 4 ou des masses à mouler thermoplastiques suivant la revendication 5, ou que l'on obtient suivant l'une quelconque des revendications 6 à 10, pour la préparation de mousses biologiquement dégradables.

**19.** Mousses biologiquement dégradables, que l'on peut obtenir par l'utilisation suivant la revendication 18.

**20.** Agents d'encollage du papier biologiquement dégradables, que l'on peut obtenir par l'utilisation des polymères biologiquement dégradables suivant les revendications 1 à 4 ou des masses à mouler thermoplastiques suivant la revendication 5 ou que l'on obtient suivant les revendications 6 à 10.